# NEUE EUROPÄISCHE PATENTSCHRIFT

(11) **EP 1 049 442 B2**
(45) Veröffentlichungstag und Bekanntmachung des Hinweises auf die Entscheidung über den Einspruch: **07.03.2007**
(45) Hinweis auf die Patenterteilung: 19.12.2001
(21) Anmeldenummer: 98920477.1
(22) Anmeldetag: 26.03.1998
(51) Int. Cl.: A61K 6/093

(54) **UNTERFÜTTERUNG FÜR PROTHESEN UND VERFAHREN ZUR HERSTELLUNG**
REBASE MATERIAL FOR PROSTHESIS AND METHOD FOR THE PRODUCTION THEREOF
MATERIAU DE REBASAGE POUR PROTHESE, ET SON PROCEDE DE PRODUCTION

(30) Priorität: 22.01.1998 DE 29800814 U
(43) Veröffentlichungstag der Anmeldung: 08.11.2000
(73) Patentinhaber: Kettenbach GmbH & CO. KG, 35713 Eschenburg (DE)
(72) Erfinder: BUBLEWITZ, Alexander, D-35745 Herborn (DE); REBER, Jens-Peter, D-35745 Herborn (DE)
(74) Vertreter: Keil, Rainer A.
(86) Internationale Anmeldenummer: PCT/EP1998/001457
(87) Internationale Veröffentlichungsnummer: WO 1999/037272

(56) Entgegenhaltungen:
- EP-A- 0 263 039
- EP-A- 0 614 655
- EP-A- 0 632 060
- EP-A- 0 826 359
- EP-A- 0 826 733
- EP-B- 0 737 721
- WO-A-91/17046
- DE-A- 3 126 663
- DE-A- 3 911 520
- US-A- 5 679 734
- DATABASE WPI Section Ch, Week 9602 Derwent Publications Ltd., London, GB; Class A26, AN 96-017100 XP002094299 & JP 07 291820 A (TOKUYAMA SODA KK) , 7. November 1995 in der Anmeldung erwähnt
- Datenblatt GE Bayer Silicones RTV-2K Siliconkautschuk,06/97
- Datenblatt Bayer Silopren U Vernetzer, 11/96
- Datenblatt Bayer AG Silopren U Katalysatoren, 07/95
- vorläufiges Datenblatt Bayer AG Silopren U Kettenverlängerer VP AI 3666, 12/96
- Datenblatt Bayer AG Silopren U, 07/95
- Datenblatt Bayer AG Silopren U Grundmischung P 1300,07/95
- Datenblatt Bayer AG Silopren S , 07/95
- vorläufiges Datenblatt Bayer AG Silopren U Vernetzer 30 VP AI 3665
- DT 2427738 A1
- DT 2644555 A1
- Hanse Chemie, Analysenzertifikat Crosslinker 101,21.03.97
- Hanse Chemie, technisches Datenblatt, 08/99
- Sicherheitdatenblatt, Crosslinker 200,26.06.97
- Datenblätter Bayer AG Silopren U, 11/96 und 07/95

## Beschreibung

### Anwendungsgebiet

Die Erfindung betrifft eine Unterfütterung für Prothesen auf der Basis von Silicon und einem Adhäsiv für einen dauerhaften Verbund von Prothesenkunststoff mit dem Silicon.

Bei der Verbindung von Siliconen mit Kunststoffen, insbesondere in der Zahnheilkunde, besteht das Problem, einen festen, dauerhaften und stabilen Verbund zu erhalten. So wird zur Aufrechterhaltung der Funküonstüchtigkeit von Zahnprothesen infolge von Veränderungen des Prothesenlagers die Zahnprothese mit einem Unterfütterungsmaterial der Unterfütterung versehen.

Harte und weichbleibende Materialien, die zur Unterfütterung einer Zahnprothese verwendet werden, sollten aus werkstoffkundlicher Sicht eine gute Haftung am Prothesenkunststoff, eine hinreichende Dimensions-stabilität, eine geringe Wasseraufnahme, eine hohe Abrasionsfestigkeit unter Mundbedingungen und eine glatte Oberfläche aufweisen. Aus klinischer Sicht kommen die Forderungen an eine Biokompatibilität, eine Geruchs- und Geschmacksneutralität, eine Widerstandsfähigkeit gegen Pilze und Bakterien, eine Farbstabilität, ein ästhetisches Aussehen und eine leichte Reinigbarkeit hinzu. Eine weitere Forderung ist die schnelle Durchführbarkeit der Unterfütterung einschließlich funktioneller Ausformung im sogenannten direkten Unterfütterungsverfahren in einem Arbeitsgang beim Zahnarzt, ohne den Umweg über das Zahntechnikerlabor.

Weiterhin ist eine gute Bearbeitbarkeit mit zahnärztlichen Instrumenten z.B. Fräsen, Skalpelle, Polierscheiben von hoher Bedeutung.

Unter guter Bearbeitbarkeit ist zu verstehen, daß das ausgehärtete Unterfütterungsmaterial mit rotierenden Instrumenten ausgearbeitet werden kann. D.h. ein Abrutschen mit der Fräse, ein Radiergummieffekt oder ein Wegbrechen größerer Siliconteile findet nicht statt.

Als Unterfütterungsmaterialien sind nach dem Stand der Technik bisher bekannt Naturkautschuk, Polyvinylverbindungen, Fluorelastomere, harte und weichbleibende Acrylsäurederivate und Silicone.

Den bekannten weichbleibenden Materialien haften Nachteile, wie geringe Lagerstabilität, ungenügende Haftung zur Prothesenbasis, Versprödung, Verfärbung u.a. an. Von den vorgeschlagenen Materialien finden für die weichbleibende Unterfütterung Acrylsäurederivate mit Weichmachern und Silicone am häufigsten Verwendung. Die allgemeinen Eigenschaften der Acrylsäurederivate sind jedoch unbefriedigend. Sie weisen aufgrund ihrer ähnlichen Zusammensetzung wie die Prothesenkunststoffe zwar eine sehr gute Anbindung und somit Haftung auf diesen auf, jedoch diffundiert der in ihnen enthaltene Weichmacher im Laufe der Zeit heraus, was zur Versprödung, Dimensionsänderung und zur Porösität des Unterfütterungsmaterials führt. Zudem können Acrylsäurederivate aufgrund der toxischen Wirkung der Monomerbestandteile auf die Schleimhaut nur eingeschränkt für die direkte Unterfütterungsmethode im Patientenmund angewendet werden. Die indirekte Unterfütterung bedingt einen labortechnischen Arbeitsgang mit eventuellen Fehlerquellen, eine zweite Behandlungssitzung und für den Patienten einen längeren Verzicht auf seine Prothese.

Für die harte Unterfütterung werden heute üblicherweise hartwerdende (Meth)acrylsäurederivate verwendet. Sie verbinden sich zwar gut mit dem Prothesenkunststoff, werden aber wegen der schon oben beschriebenen Nachteile fast ausschließlich für das indirekte Unterfütterungsverfahren eingesetzt. Außerdem wäre beim Einsatz dieser Materialien im direkten Unterfütterungsverfahren die problemlose Mundentnahme, z.B. bei Teilprothesen durch das Einfließen von Material in unter-sich-gehende Stellen gefährdet.

In Bezug auf die Anforderungen eines Unterfütterungsmaterials im direkten Verfahren sind die Silicone am ehesten geeignet. Jedoch erweist sich deren Haftung am Prothesenkunststoff aus Polymethylmethacrylat als äußerst unbefriedigend. Eine Verminderung dieses unerwünschten Effektes wird nach dem Stand der Technik durch mechanische Retentionen und die Verwendung von Haftvermittlern erreicht, die aus einer Mischung von Acryloxyalkylsilanen, z.B. Methacryloxypropyltrimethoxysilan bestehen. Die damit erzielbaren Verbundfestigkeiten liegen aber noch immer unter 100 Ncm⁻².

Aus der DE-A- 39 02 817 ist bekannt, daß die Haftung von additionsvernetzenden Siliconen am Prothesenkunststoff durch Zusatz von 10 bis 50 Gew.% pulverförmigen Methacrylat(co)polymer als Füllstoff zu einer wärmehärtenden Vinyisiliconmasse des Additionspolymerisationstyps zu Bindefestigkeiten von bis zu 200 Ncm⁻² führt. Durch den hohen Anteil von Methacrylat-Kunststoff gehen jedoch die typischen für Unterfütterungsmaterialien wichtigen Siliconeigenschaften, wie Dauerelastizität und wasserabweisende Wirkung, teilweise wieder verloren, so daß für ein solches Unterfütterungsmaterial nur eine Haltbarkeit von sechs Monaten angegeben werden kann.

Die DE-A-39 11 520 beschreibt eine dauerhaft haltbare und weichbleibende Unterfütterung auf der Basis von Siliconkautschuk. Die Haftung-zwischen Prothesenkunststoff und Silicon wird hier durch Aufvulkanisieren des Silicons auf eine Stahlarmierung bei 200°C und anschließende Aufbringung des Prothesenkunststoffes und der Zähne auf die Stahlbasis erzielt. Nachteilig bei diesem Verfahren sind die hohen erforderlichen Temperaturen und die benötigte aufwendige apparative Ausstattung.

In PCT/EP 950 13 13 und DE-A-195 39 653 werden 2-Komponenten-Polyurethan-Adhäsive mit einem Zusatz von Organohydrogenpolysloxanen für den dauerhaften Verbund von Prothesenkunststoffen mit additionsvernetzenden Siliconen beschrieben.

Hiermit lassen sich zwar gute Haftverbunde erzielen, jedoch mit allen bekannten Nachteilen, die ein 2-Komponenten-System gegenüber einem 1-Komponenten-System mit sich bringt: höherer Zeitaufwand zum homogenen Mischen der beiden Komponenten, und ein höherer Zeitaufwand durch die erforderlichen Reaktionszeiten der beiden miteinander reagierenden Komponenten, Gefahr von Mischinhomogenitäten, fehlerhafter Dosierung und Einmischen von Luft.

Weiterhin besteht bei 2-Komponenten-Polyurethan-Systemen die Gefahr des Schäumens bzw. Gasen, abhängig von der Luftfeuchtigkeit in der Umgebung.

In EP-A- 0 632 060 wird die Verwendung von siliconmodifizierten Acryl-Copolymerisaten, mit mittleren Molekulargewichten zwischen 5.000 und 1.000.000 g/mol, als Einkomponenten-Adhäsiv (Bestandteil von Handelsprodukt 1, siehe Tabelle 1 - 4) beschrieben, das durch Copolymerisation von Methylmethacrylat mit Allylmethacrylat und anschließender platinkatalysierter Hydrosilylierung des entstandenen allylgruppenhaltigen Copolymers mit einem Hydrogenpolydimethylsiloxan hergestellt wird.

Der Syntheseaufwand für dieses Adhäsiv ist jedoch hoch.

Zum einen stören die Katalysatoren der Copolymerisation die platinkatalysierte Hydrosilylierung und zum anderen muß der verbrauchte Platinkatalysator nach der Hydrosilylierung aus dem Produkt entfernt werden, um Stabilitätsprobleme durch Wasserstoffentwicklung zu verhindern.

Die mit diesem Adhäsiv erzielbaren Haftkräfte führen zu einem Verbund zwischen PMMA-Kunststoff und dem zugehörigen Unterfütterungssilicon (Bestandteil von Handelsprodukte 1) nach EP-A- 0 614 655.

Die im Zugversuch ermittelten Werte für die Bruchspannung dieses Haftverbundes liegen zwischen 67 und 75 N/cm² (siehe Tabelle 2a). Das Bruchbild zeigt zwar einen reinen Kohäsionsbruch im Silicon, allerdings ist aus der oben genannten Bruchspannung zu erkennen, daß die Reißfestigkeit des Unterfütterungssilicons nach EP-A- 0 614 655 (Bestandteil von Handelsprodukt 1) vergleichsweise niedrige Werte besitzt, so daß eine hohe Reiß- und Weiterreißfestigkeit für das eingesetzte Unterfütterungssilicon nur unzureichend gewährleistet ist, um der Dauerbelastung der Kaubewegung bei einer Langzeitunterfütterung standzuhalten.

Weiterhin zeigt das Unterfütterungssilicon des Handels-produkts 1, basierend auf EP-A- 0 614 655, eine sehr hohe (nicht anwenderfreundliche) Handkraft beim Austragen aus einer Kartusche. (Siehe Tabelle 1, vergleichende Untersuchungen.)

Denn günstigerweise wird das Unterfütterungssilicon mittels einer Handaustragpistole aus einer Kartusche mit einem statischen Mischer dosiert und gemischt. Hierbei ist es wichtig, daß die erforderliche Handkraft, die zum Austragen des Unterfütterungssilicons benötigt wird, so gering wie möglich ist, um eine anwenderfreundliche Bedienung zu gewährleisten.

Das Unterfütterungssilicon gemäß EP-A- 0 614 655 (siehe Handelsprodukt 1) zeigt keine ausreichend lange Verarbeitungszeit unter den genannten Bedingungen, so daß hier die mimischen Bewegungen des Patienten nur unzureichend abgebildet werden können. Für eine direkte Unterfütterung der Prothese in der Zahnarzt-praxis (chairside) ist jedoch eine ausreichend lange Verarbeitungszeit des Unterfütterungsmaterials unter Mundbedingungen (35°C) erforderlich.

Zudem kann dieses Unterfütterungssilicon (siehe Handelsprodukt 1) aufgrund seiner geringen Standfestigkeit während der Funktionsabformung in den Rachenraum des Patienten einfließen.

Für die Funktionsabformung ist jedoch eine hohe Standfestigkeit des Unterfütterungssilicons während der Verarbeitungszeit erforderlich, um ein unerwünschtes Herausfließen des Materials aus dem zu unterfütternden Bereich zu verhindern. Weiterhin besteht die Gefahr, daß die Schichtdicke der Unterfütterung zu gering ausfällt, um Paßgenauigkeit und Tragekomfort der unterfütterten Prothese zu gewährleisten.

Die Reiß- und Weiterreißfestigkeit sowie die Elastizität ist auch für den nach JP 07291820 A anzuwendenden 2-Komponenten-Unterfütterungssilicon-Versiegelungslack, auch "coat", "politur", "glazing" genannt, nicht ausreichend. Dieser zeigt nach der Vulkanisation eine unzureichende Elastizität und ist sehr spröde. (Siehe Tabelle 4, vergleichende Untersuchungen.)

Auf dem Markt existieren zur Zeit weitere Unterfütterungssysteme, die die gestellten Anforderungen an das Gesamtsystem nur mit Einschränkungen erfüllen.

So ist das Unterfütterungsmaterial des Handels-produktes 2 (siehe Tabelle 1 bis 4) zu dünnfließend, um eine korrekte Funktionsabformung durchführen zu können. Eine komfortable, paßgenaue Unterfütterung ist mit diesem Material aus oben genannten Gründen schwer herstellbar.

Nach Verarbeitung gemäß der vom Hersteller ausgewiesenen Packungsbeilage ist der erforderliche Haftverbund durch das zugehörige Unterfütterungsadhäsiv zwischen Prothesenkunststoff und Unterfütterungssilicon nach Ende der angegebenen Abbindezeit nicht vollständig ausgebildet. Abscherversuche (Tabelle 2a/ 2b, vergleichende Untersuchungen) zeigen anhand des Bruchbildes, daß kein reiner Kohäsionsbruch im Unterfütterungssilicon vorliegt, sondern daß ein Teil des Haftverbundes zwischen Unterfütterungssilicon und Prothesenkunststoff gelöst ist.

Bei einem Bearbeiten der Unterfütterung nach der angegebenen Abbindezeit mit einem Skalpell oder einer Fräse besteht somit die Gefahr, daß der Haftverbund zwischen Unterfütterungssilicon und Prothesenkunststoff irreversibel geschädigt wird und die betroffenen Bereiche zu Ausgangspunkten für den Angriff von Wasser, Bakterien und Pilzen unter Mundbedingungen werden, so daß eine solche Unterfütterung der Dauerbelastung einer langzeitigen Anwendung nur unzureichend standhalten kann. Der 2-Komponenten-Versiegelungslack von Handelsprodukt 2 zeigt nach der Vulkanisation eine hohe Eigenklebrigkeit und eine vergleichsweise geringe Reiß- und Weiterreißfestigkeit (siehe Tabelle 4).

Untersuchungen zum Handelsprodukt 3 (siehe Tabelle 1 bis 4) nach den Vorgaben der Packungsbeilage, zeigen eine unzureichende Abstimmung zwischen dem Abbindeverhalten des Unterfütterungssilicons und der Ausbildung des Haftverbundes zwischen Unterfütterungssilicon und Prothesenkunststoff.

Nach der angegebenen Muridverweildauer von 5 min. hat sich der Haftverbund zwischen Unterfütterungssilicon und Prothesenkunststoff noch nicht vollständig ausgebildet. Nach Zugversuchen zeigt das Bruchbild, daß sich der Verbund zwischen Prothesenkunststoff und Unterfütterungssilicon teilweise trennt. Eine auf diese Weise unterfütterte Prothese kann die Anforderungen einer langzeitigen Dauerbelastung unzureichend erfüllen, da nach kurzer Zeit unter Mundbedingungen mit einem Eindringen von Wasser, Verunreinigungen, Bakterien und Pilzen zwischen Prothesenkunststoff und Unterfütterungssilicon und in der Folge mit einem Ablösen der Unterfütterung von derProthesenbasis zu rechnen ist.

Die Anfertigung einer Unterfütterung beim Zahnarzt oder beim Zahntechniker sollte in angemessener Zeit durchführbar sein. Beim Unterfütterungs-Versiegelungslack des Handelsproduktes 3 benötigt die Aushärtung ca. 30 min. bei Raumtemperatur, wodurch die Wartezeit des Patienten bzw. die Arbeit des Zahnarztes oder Zahntechnikers unnötig verlängert ist.

Zudem zeigt der 2-Komponenten-Unterfütterungs-Versiegelungslack nach der Vulkanisation eine unzureichende Elastizität und ist sehr spröde (s. Tabelle 4).

Die DE-A- 19 614 983 beschreibt ein weiches Unterfütterungsmaterial für Prothesen, welches sich nicht von der Prothese trennt. Aus dieser Druckschrift geht jedoch nicht hervor, auf welche Weise der Haftverbund zwischen Prothesenkunststoff und Unterfütterungssilicon hergestellt wird.

Vergleichsversuche zeigen jedoch, daß Unterfütterungs-silicone des beschriebenen Typs ohne Unterfütterungsadhäsiv nicht auf Prothesenkunststoff haften. Zugversuche zeigen einen mäßigen Haftverbund mit einem Bruchbild, das einen nicht kohäsiven Bruch zwischen Unterfütterungssilicon und Prothesenkunststoff aufweist. Eine nach DE 19 614 983 A1 unterfütterte Prothese kann einer langzeitigen Dauerbelastung nur bedingt standhalten.

Es ist daher Aufgabe der Erfindung, eine Unterfütterung für Prothesen und ein Verfahren zu dessen Herstellung zu schaffen, das hinsichtlich der Leistungsfähigkeit in den Punkten Langzeitstabilität, hohe Reißfestigkeit und Weiterreißfestigkeit und damit einer guten Bearbeitbarkeit mit zahnärztlichen Instrumenten, wie Fräsen, Skalpelle, Polierscheiben und ein sofort zur Verfügung stehender hoher Haftverbund zum Prothesenkunststoff sowie ein hoher Haftverbund zu anderen zahnärztlichen Werkstoffen wie Metallen, Metallegierungen, Keramiken und Gläsern und hinsichtlich der anwendungstechnischen Eigenschaften der Einzelkomponenten, wie Fließverhalten, d.h. Viskosität, Standfestigkeit, Ausdrückbarkeit, und Abbindecharakteristik, d.h. Verarbeitungszeit, Abbindeende gegenüber dem Stand der Technik verbessert ist.

Diese Aufgabe wird durch die Merkmale der Ansprüche 1, 4 und 9 gelöst.

Dazu ist es erfindungsgemäß vorgesehen, eine Unterfütterung zu schaffen, die aus einem 1-Komponenten-Unterfütterungsadhäsiv, einem additionsvernetzenden 2-Komponenten-Unterfütterungssilicon und einem additionsvernetzenden 2-Komponenten Versiegelungslack besteht.

Das 1-Komponenten-Unterfütterungsadhäsiv besteht aus einem erfindungsgemäßen Gemisch, das aus (Meth)acrylsäureestern und einem funktionalisierten Silan mit Methacrylat und anderen ungesättigten Gruppen hergestellt wird, und bereits bei Raumtemperatur schnell einen hohen und stabilen Haftverbund zwischen Prothesenkunststoff und Unterfütterungssilicon bzw. Unterfütterungs-Versiegelungslack herstellt.

Das additionsvernetzende 2-Komponenten-Unterfütterungssilicon enthält Organohydrogenpolysiloxane mit einem hohen SiH-Gehalt, die zu einem sehr hohen Haftverbund mit der erfindungsgemäßen Funktionseinheit "Unterfütterungsadhäsiv" führt (siehe Tabelle 2a, 2b, 3) und Organohydrogenpolysiloxane mit vergleichsweisen geringen SiH-Gehalt, die zu einer hohen Elastizität und Reißfestigkeit des Unterfütterungssilicons und somit erst eine gute mechanische Bearbeitbarkeit, z.B. mit rotierenden zahnärztlichen instrumenten, ermöglichen (siehe Tabelle 1).

Der additionsvernetzende, lösungsmittelfreie 2-Komponenten-Unterfütterung-Versiegelungslack zeichnet sich bei einer sehr niedrigen Viskosität (pinselbar) durch eine hohe Haftkraft zum Unterfütterungsadhäsiv und zum Unterfütterungssilicon aus, bedingt durch den Einsatz der Organohydrogenpolysiloxane mit einem hohen SiH-Gehalt, und Organohydrogenpolysiloxanen mit vergleichsweise geringem SiH-Gehalt, die zu einer vergleichsweise hohen Elastizität und Reißfestigkeit führen (siehe Tabelle 4).

Trotz der sehr niedrigen pinselbaren Mischungsviskosität, die ohne Einsatz von Lösungsmitteln erzielt wird, kann der Versiegelungslack aus Doppelkammerkartuschen mit statischem Mischer ausgetragen und homogenisiert werden, so daß ein Einschluß von Luftblasen ausgeschlossen ist.

Die erfindungsgemäße Unterfütterung löst die Aufgabe unter Berücksichtigung der sonstigen mechanischen und verarbeitungsfreundlichen Eigenschaften hinsichtlich sinnvoll verarbeitbarer Viskositäten/Konsistenzen mit den gängigen Dosier- und Mischsystemen (siehe Tabelle 1), praxisorientiertem Abbindeverhalten, Bearbeitbarkeit, Standfestigkeit und Fließverhalten während der Verarbeitungszeit bei der Funktionsabformung und glatter Oberflächenbeschaffenheit des ausgehärteten Unterfütterungssilicons/Unterfütterungs-Versiegelungslacks eine verbesserte Unterfütterung zu erreichen.

Weiterhin verfügt die Formulierungen der Inhaltsstoffe über eine ausreichende Lagerstabilität in der entsprechenden Primärverpackung, z.B. Kartusche, Tube oder Flasche.

Das erfindungsgemäße Einkomponenten-Unterfütterungsadhäsiv ist überall dort einsetzbar, wo Silicone mit Kunststoffen, Metallen, Metallegierungen, Keramiken und Gläsern verklebt werden sollen.

Das Einkomponenten-Unterfütterungsadhäsiv ist bevorzugt in der Zahnheilkunde und Epithetik anzuwenden.

Das 1-Komponenten-Unterfütterungsadhäsiv verbessert die Verbundfestigkeit zwischen Silicon und Prothesenkunststoff unter Raumtemperaturbedingungen in einem besonders hohen Maße, indem es nach Auftragen auf einen Prothesenkunststoff in dünner Schicht einen hohen Haftverbund zu dem erfindungsgemäßen Unterfütterungssilicon bzw. Unterfütterungssilicon-Versiegelungslack aufweist. Somit werden die vorteilhaften Eigenschaften der Silicone als Unterfütterungsmaterialien nutzbar gemacht.

Ein weiterer Vorteil des erfindungsgemäßen 1-Komponenten-Unterfütterungsadhäsiv ist, daß es auch zum Verkleben von Prothesen verwendet werden kann, die neben Prothesenkunststoff metallische (Legierungen) oder keramische Bauteile enthalten. Hierzu wird die mit Adhäsiv versehene Prothese einige Minuten bei Temperaturen > 30°C getempert.

Dadurch wird ein guter Haftverbund zwischen den metallischen und/oder keramischen Bauteilen und dem Silicon geschaffen.

Es ist somit vorgesehen, ein 1-Komponenten-Unter-fütterungsadhäsiv zu schaffen, das zum Verkleben von Kunststoffen, insbesondere von PMMA mit Silicon geeignet ist und einen guten Haftverbund sowohl zum (Prothesen)-Kunststoff als auch zum Silicon schafft.

Besonders vorteilhaft ist das Aufbringen des erfindungsgemäßen Unterfütterungsadhäsivs in gelöster Form auf den zu verklebenden (Prothesen)-Kunststoff, da das Lösungsmittel, je nach Flüchtigkeit, schnell abdampft (z.B. Dichlormethan 90 sec.) und somit in sehr kurzer Zeit eine konditionierte (Prothesen)-Kunststoff-Oberfläche zur Verfügung steht, auf die ohne weitere Wartezeit das erfindungsgemäße Unterfütterungssilicon aufgebracht werden kann.

Ein weiterer Vorteil besteht darin, daß sofort nach Aushärten des erfindungsgemäßen Unterfütterungssilicons auf dem mit dem erfindungsgemäßen Unterfütterungsadhäsiv konditionierten (Prothesen)-Kunststoff-Oberfläche, ein so starker Haftverbund erzielt wird, der ein zügiges, praxisgerechtes Bearbeiten, z.B. mit einer Fräse, ermöglicht.

Auf diese Weise ist eine schnelle und effiziente Unterfütterung der Zahnprothese sowohl direkt beim Zahnarzt als auch indirekt im Zahnlabor möglich.

Untersuchungen zeigen, daß das erfindungsgemäße Unterfütterungsadhäsiv mit allen gängigen Prothesenkunststoffen, z.B. Kaltpolymerisaten, Heißpolymerisaten und Spritzgußprothesenkunststoffen dauerhafte, hohe Haftverbunde gewährleistet; siehe Tabelle 2b.

Die Strukturmerkmale der erfindungsgemäßen AdhäsivKomponente Z setzen sich wie folgt zusammen:
a) - ethylenisch ungesättigte Verbindung wie z.B. vom Typ 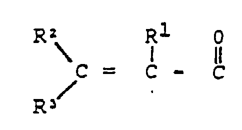 R¹: H, Halogen, Alkyl, substituiertes Alkyl, CN
   R², R³: H, Halogen, Alkyl, substituiertes Alkyl
   z.B.:
   - α-Methylvinylcarbonyl-
   - β -Methylvinylcarbonyl-
   - Vinylcarbonyl
   - Vinyl
b) B = O, S, NH, NR, PH, PR
c) A = Spacer, geradkettigen oder verzweigten Kohlenwasserstoffrestes mit 0 bis 30-C Atomen, der auch einen aromatisch ungesättigten oder cycloaliphatischen Ring enthalten kann
d) Polydialkylsiloxy-: z.B.: Polydimethylsiloxy vom Typ 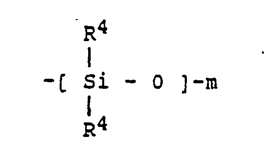
e) ein Siloxan, enthaltend eine trifunktionelle T- und/ oder eine tetrafunktionelle Q-Einheit
wobei R⁴ in Pkt. d) und e) eine ungesättigte Gruppe darstellt

z.B. Alkenyl, Alkinyl, Alkenyloxy, Alkinyloxy, z.B. Vinyloxy und Allyloxy, wobei die oben genannten Gruppen sowohl cyclisch, linear als auch verzweigt vorliegen können und/oder eine der folgenden Gruppen:

Alkyl- (z.B. Methyl-, Ethyl-, Isopropyl-), Aryl-(z.B. Phenyl-, Naphtyl-, Tolyl-, Xylyl-), Aralkyl(Benzyl-, Phenylethyl-) und halogensubstituierte Alkyl- und Aryl-Gruppen (z.B. 3,3,3,-Trifluorpropyl-, Chlorphenyl-, Difluorphenyl-), Cyanalkyl-, Cycloalkyl-
wobei R⁵ gleich R⁴ oder R⁶ sein kann,
wobei R⁶ sich zusammensetzt aus den unter 2.1 a) bis d) beschriebenen Struktureinheiten.

Die Strukturmerkmale aus den Punkten a) bis e) setzen sich zur folgenden allgemeinen Formel zusammen: 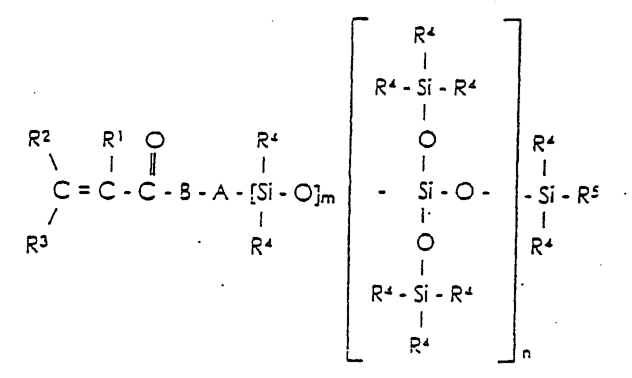 wobei n und m ganze Zahlen im Bereich von Null bis 1000 sind.

Das erfindungsgemäße 1-Komponenten-Unterfütterungs-adhäsiv kann in Form der reinen Adhäsivkomponente Z oder als Copolymer, beinhaltend die Adhäsivkomponente Z, oder als Mischung des Copolymers mit den Ausgangs-stoffen (Adhäsivkomponente Z, Monomere) eingesetzt werden.

Das Copolymer wird beispielsweise wie folgt beschrieben synthetisiert:
die Adhäsivkomponente Z wird mit geeigneten Monomeren, wie Acrylat- bzw. Methacylat polymerisiert.

Als Beispiele für derartige Monomere, die polymerisiert werden können, seien aufgeführt:

Methacrylate und Acrylate, vorzugsweise mono- oder polyfunktionelle Methacrylate, insbesondere Alkylmethacrylat, Cycloalkylmethacrylat, Isoalkylmethacrylat, Tetraethylenglycoldimethacrylat, Triethylenglycoldimethacrylat, Diethylenglycoldimethacrylat, Ethylenglykoldimethacrylat, Polyethylenglykoldimethacrylat, Butandioldimethacrylat, Hexandioldimethacrylat, Decandioldimethacrylat, Dodecandioldimethacrylat, Bisphenol-A-dimethacrylat, Trimethylolpropantrimethacrylat, 2,2-Bis-4(3-methacryloxy-2-hydroxy)-phenylpropan(Bis-GMA) sowie Reaktionsprodukte aus Di- und/oder Triisocyanaten und OH-gruppenhaltigen Methacrylaten.

Die Reaktion kann als Kalt-, Heiß- oder Lichtpolymerisation durchgeführt werden.

Als Katalysator für die Heißpolymerisation des ethylenisch ungesättigten Monomeren kommen die üblichen Katalysatoren in Frage, beispielsweise Peroxide wie Dibenzoylperoxid, Dilauroylperoxid, tert.-Butylperoctoat, tert.-Butylperbenzoat, aber auch α, α'-Azo-bis-(isobutyroethylester), AIBN, Benzpinakol und 2,2'-Dimethylbenzpinakol.

Als Katalysator für die Kaltpolymerisation des ethylenisch ungesättigten Monomeren kommen die üblichen in Frage, insbesondere Amin/Peroxid-Systeme (z.B. DibenzoylperoxidlN,N-Dimethyl-p-toluidin).

Als Katalysator für die Lichtpolymerisation des ethylenisch ungesättigten Polymeren eignen sich Benzophenon und seine Derivate, Benzoin und seine Derivate, Acylphospinoxide, insbesondere α-Diketone wie Campherchinon, gegebenenfalls in Verbindung mit einem Amin als Reduktionsmittel.

Als Polymerisationsverfahren finden folgende Verfahren Anwendung:

Massepolymerisation, Lösungspolymerisation (z.B. Toluol, Chloroform, Methylenchlorid), Fällungspolymerisation (in reinen Lösungsmitteln oder Lösungsmittelgemischen wie z.B. Methylenchlorid/ Hexan) und Suspensionspolymerisation (z.B. Wasser mit Tensiden, Emulgatoren).

Das erfindungsgemäße 1-Komponenten-Unterfütterungs-adhäsiv, kann in Form der reinen Adhäsivkomponente Z oder als Copolymer, beinhaltend die Adhäsivkomponente Z, oder als Mischung des Copolymers mit den Ausgangsstoffen (Adhäsivkomponente Z, Monomere) verwendet werden.

Folgende Zusammensetzung wird vorzugsweise verwendet:

In gelöster Form

| | |
|---|---|
| a) Adhäsivkomponente Z als Reinsubstanz | 0 bis 99 Gew.% |
| b) Copolymer mit der Adhäsivkomponente Z als Bestandteil | 0 bis 99 Gew.% |
| c) Copolymer in der Mischung mit den Ausgangsstoffen (Adhäsivkomponente Z, Monomer) | 0 bis 99 Gew.% |
| wobei die Summe der Gewichtsanteile der Bestandteile a), b) und c) mindestens | 0,01 Gew.% ist. |
| d) Katalysatoren zur Beschleunigung der Ausbildung des Haftverbundes zum zuverklebenden Silicon | 0 bis 10 Gew.% |
| e) Lösungsmittel | 1 bis 99,99 Gew.% |
| f) Farbstoffe | 0 bis 20 Gew.% |
| g) Tenside, Emulgatoren und Stabilisatoren | 0 bis 20 Gew.% |
| h) Organohydrogensiloxan | 0 bis 40 Gew.% |
| i) α-, ω-Divinylpolydialkylsiloxan | 0 bis 40 Gew.% |

Die Adhäsivkomponente Z als Reinsubstanz a) setzt sich gemäß oben beschriebener allgemeiner Formel zusammen.

Das Copolymer b), das die Adhäsivkomponente Z enthält, wird wie oben beschrieben durch Polymerisation hergestellt.

Die Mischung c) des Copolymers mit den Ausgangsstoffen (Adhäsivkomponente Z und Monomer) besteht aus folgenden Bestandteilen:
- dem Copolymer 0,1 bis 99,9 Gew.%
- der Adhäsivkomponente Z 0,1 bis 99,9 Gew.%
- dem Monomer 0,1 bis 99,9 Gew.%
wobei die Summe der drei Bestandteile 100% ergibt.

Als Katalysatoren zur Beschleunigung der Ausbildung des Häftverbundes zum zu verklebenden Silicon Id) dienen Salze, Komplexe und kolloidal vorliegende Formen der Übergangsmetalle der 8. Nebengruppe, vorzugsweise die der Metalle Platin, Palladium und

Rhodium, insbesondere erweisen sich Platinkomplexe, die aus Hexachloroplatinsäure hergestellt werden, als geeignet und radikalbildende Stoffe, wie Peroxide, vorzugsweise Diaroyl-, Dialkyl-, Diaralkyl-, Alkylaralkyl-, Alkylaroyl-peroxide und Schwefel.

Als Lösungsmittel e) können alle organischen Lösungsmittel wie Aromaten, Benzine, Ether, Ester, Alkohole, Ketone, Aldehyde, Amine, Carbonsäuren, halogenierte Lösungsmittel z.B. Chloroform und Wasser sowie die möglichen Kombinationen aus oben genannten Lösungsmitteln eingesetzt werden.

Bei den unter f) genannten Farbstoffen handelt es sich um lösliche Farbstoffe oder um Pigmentfarbstoffe. Im Bereich der zahnmedizinischen und medizinischen Anwendungen des Klebstoffs werden ausschließlich Lebensmittelfarbstoffe, die vom BGA als solche zugelassen sind, eingesetzt. Zur Verwendung gelangen gleichfalls Farbpasten aus Polysiloxan-, Mineralöl-, Lebensmittelfarbstoff-Formulierungen.

Bei den als Emulgatoren und Stabilisatoren eingesetzten Komponenten g) handelt es sich um anionische Tenside, insbesondere Alkylsulfate, Alkylbenzolsulfonate und -phosphate, kationische Tenside, insbesondere Tetraalkylammoniumhalogenide, -sulfate oder -acetate oder Alkylpyridiniumhalogenide, nichtionische Tenside, insbesondere Alkyl- und Alkyl-phenyl-Polyethylenglycole und -glycolether, Fettsäurealkylolamide, Saccharosefettsäureester, Trialkylaminoxide, Silicontenside oder Fluortenside sowie amphotere Tenside, insbesondere sulfatierte oder oxyethylierte Kondensationsprodukte aus Alkylphenolen und Formaldehyd, Ethylenoxid-Propylenoxid-Blockpolymerisate, modifizierte Polysiloxane, Polyvinyl-N-alkylpyridiniumsalze oder Copolymerisate aus Vinylpyridin und Methacrylsäure. Die Tenside können auch funktionelle Gruppen, wie z.B.
-OH,-CH=CH₂,-OCO-(CH₃)C=CH₂, SiH und -Si-CH=CH₂ enthalten.

Die Organohydrogensiloxan-Komponenten h) fungieren als Vernetzer und sind Polyalkyl- und -aryl-Siloxane sowie Polyhalogenalkyl- und -aryl-Siloxane, welche im Molekül mindestens zwei an Siliciumatome gebundene Wasserstoffatome aufweisen. Verwendet werden Vernetzer mit einem SiH-Gehalt von 1 bis 15 mmol/g, vorzugsweise mit 2 bis 11 mmol/g.

Die Organopolysiloxane i) mit zwei Vinylgruppen im Molekül haben eine Viskosität zwischen 21 und 200.000 mPa·s.

Bei dem Epithedk-/Unterfütterungssilicon handelt es sich um ein additionsvernetzendes Silicon, besehend aus zwei Komponenten, die über eine durch edelmetallkatalysierte Hydrosilylierungsreaktion chemisch vernetzen.

Die Komponenten A und B sind in verschiedenen Viskositäten (dünn-, mittel-, zähfließend und knetbar) und Mischungsverhältnissen (in der Regel 1:1) einstellbar. Die Mischung der Komponenten A und B ist gewichts-und volumenmäßig durchführbar und erfolgt über geeignete Dosier- und Mischvorrichtungen, wie z.B. Stranglängendosierung und manuelles Mischen, Kartuschensystem mit statischem Mischer und elektrisch/ mechanisch betriebene Mischgeräte. Nach dem Dosieren und Mischen der Komponenten A und B wird die resultierende, homogen gemischte Paste innerhalb der Gesamtverarbeitungszeit mit geeigneten Hilfsmitteln in den Patientenmund appliziert. Anschließend führt der Patient Funktionsbewegungen durch, die während der Verarbeitungszeit von dem Unterfütterungssiliconmaterial, das auch gleichzeitig die Aufgabe der Funktionsabformung übernehmen kann, registriert werden.

Nach dem Aushärten des Materials (Mundverweildauer) wird dieses aus dem Patientenmund entnommen und nach Bedarf weiterverarbeitet.

Das erfindungsgemäße Siliconmaterial zeichnet sich gegenüber dem Stand der Technik insbesondere durch eine hohe Reißfestigkeit und durch einen ausgezeichneten Haftverbund zum erfindungsgemäßen Unterfütterungsadhäsiv im vulkanisierten Zustand aus.

Überraschenderweise hat sich gezeigt, daß die Kettenlänge, der SiH-Gehalt und die Konzentration des Organohydrogenpolysiloxans einen großen Einfluß auf die Haftkraft zum erfindungsgemäßen 1-K-Unter-fütterungsadhäsiv und auch auf die Reißfestigkeit haben.

Weiterhin zeichnet sich das erfindungsgemäße Unterfütterungssilicon neben einer praxisgerechten Verarbeitbarkeit und einem guten Fließverhalten während der Verarbeitungszeit durch ein patientenfreundliches Abbinden im Mund und durch eine ausreichend lange Verarbeitungszeit im Mund, damit der Patient seine Funktionsbewegungen durchführen kann, aus.

Des weiteren realisiert die erfindungsgemäße Zusammensetzung des Siliconmaterials auf Basis von Organopolysiloxanen mit zwei Vinylgruppen im Molekül und einer Viskosität von 21 bis 200.000 mPa·s sowohl ein weichbleibendes (niedrige Shore Härte), als auch ein sehr hartes (hohe Shore Härte) Unterfütterungssilicon. Das harte Unterfütterungssilicon zeigt trotz des hohen Vinylgehaltes keine inhibierende Wirkung auf die Vulkanisationsgeschwindigkeit. In Verbindung mit dem SiH-Vernetzer und Platinkatalysator werden Vulkanisationen mit einer anwenderfreundlichen Abbindecharakteristik realisiert.

Aufgrund des hohen Haftverbundes ist eine gezielte Bearbeitbarkeit (z.B. mit Skalpell, Fräse) des im Mund ausgehärteten überschüssigen Siliconmaterials durchführbar, so daß das Unterfütterungssilicon exakt auf die individuelle Mund- und Kieferkonturen angepaßt werden kann, ohne daß sich das Unterfütterungssilicon beim Bearbeiten von Haftverbund zur Zahnprothese löst (siehe Beispiele 3, 4, 5 und 8).

Weiterhin zeigt das erfindungsgemäße Siliconmaterial nach Wasserlagerung keinen Abfall des Haftverbundes zum erfindungsgemäßen 1-Komponenten-Unterfütterungs-adhäsiv (siehe Tabelle 3).

Die hierfür geforderten Reißfestigkeiten und Anforderungen an den Haftverbund unter feuchten Mundbedingungen werden unter Beibehaltung der sonstigen mechanischen und verarbeitungsfreundlichen Eigenschaften hinsichtlich sinnvoll verarbeitbarer Viskositäten/ Konsistenzen mit den oben aufgeführten Dosier- und Mischsystemen, praxisorientiertem Abbindeverhalten, Flexibilität, Bearbeitbarkeit, Entformbarkeit, Fließverhalten während der Verarbeitungszeit und ggf. glatter Oberflächenbeschaffenheit, erzielt. Dabei verfügt die Formulierung der Inhaltsstoffe über eine ausreichende Lagerstabilität in der entsprechenden Primärverpackung, z.B. Kartusche oder Tube, wobei unter Lagerstabilität in diesem Zusammenhang beispielsweise eine Aufrechterhaltung der Leistungsmerkmale vor und während der Vulkanisation hinsichtlich der Viskosität/Konsistenz der Einzelkomponenten und der Mischung sowie der Abbindecharakteristik über mindestens 12 Monate und nach der Vulkanisation hinsichtlich der Reißfestigkeit und Haftkraft über mindestens 12 Monate zu verstehen ist.

Das erfindungsgemäße Unterfütterungssilicon weist folgende Zusammensetzung von Inhaltsstoffen und folgende Mengen der einzelnen Komponenten a) bis o) in Gew.%, bezogen auf das gesamte Siliconmaterial auf:
a1) 0,1 bis 80 Gew.%, vorzugsweise 1 bis 40 Gew.% Organohydrogenpolysiloxane und deren Mischungen und einem SiH-Gehalt von 2,00 - 15 mmol/g, vorzugsweise 3,0 - 11,0 mmol/g, mit einer Viskosität von 10 bis 10000 mPa·s, vorzugsweise von 30 bis 500 mPa·s.
a2) 0,1 bis 80 Gew.%, vorzugsweise 1 bis 40 Gew.% Organohydrogenpolysiloxane gemäß Anspruch 4 und deren Mischungen und einem SiH-Gehalt von 0,03 - 2,0 mmol/g, mit einer Viskosität von 10 bis 70000 mPa·s, vorzugsweise von 30 bis 2000 mPa·s.
b) 1 bis 90 Gew.%, vorzugsweise 10 bis 60 Gew.% Organopolysiloxane mit zwei Vinylgruppen im Molekül und einer Viskosität von 21 bis 200.000 mPa·s.
c) 0,0001 bis 1,0 Gew.% vorzugsweise 0,0005 bis 0,2 Gew.% der Katalysatoren zur Beschleunigung der Hydrosilylierungsreaktion, bezogen auf reines Metall
d) 0 bis 80 Gew.%, vorzugsweise 0 bis 50 Gew.% der verstärkenden Füllstoffe
e) 0 bis 90 Gew.%, vorzugsweise 20 bis 80 Gew.% der nicht versärkenden Füllstoffe
f) 0 bis 10 Gew.%, vorzugsweise 0 bis 2 Gew.% der Farbstoffe
g) 0 bis 50 Gew.%, vorzugsweise 0 bis 10 Gew.% der Feuchtigkeitsbinder
h) 0 bis 70 Gew.%, vorzugsweise 0 bis 20 Gew.% der Organopolysiloxane mit mehr als zwei Vinylgruppen pro Molekül
i) 0 bis 1,0 Gew.%, vorzugsweise 0 bis 0,1 Gew.% der Inhibitoren
j) 0 bis 90 Gew.%, vorzugsweise 0 bis 50 Gew.% des vinylgruppenhaltigen festen oder flüssigen MQ-Siliconharzes
k) 0 bis 80 Gew.%, vorzugsweise 0 bis 50 Gew.% eines Compounds aus vinylgruppenhaltigen Organopolysiloxanen und verstärkenden Füllstoffen
l) 0 bis 10 Gew.%, vorzugsweise 0 bis 5 Gew.% der Tenside, Emulgatoren und Stabilisatoren
m) 0 bis 90 Gew.%, vorzugsweise 0 bis 80 Gew.% der radioopaken Substanzen
n) 0 bis 20 Gew.%, vorzugsweise 0 bis 10 Gew.% der H₂-Absorber/Adsorber oder der die H₂-Entwicklung reduzierenden bzw. eliminierenden Substanzen
o) 0 bis 50 Gew.%, vorzugsweise 0 bis 35 Gew.% der Weichmacher, Neutralöle und Verteileröle.

Das erfindungsgemäße Unterfütterungssilicon beinhaltet Organohydrogenpolysiloxane a1) mit einem hohen SiH-Gehalt (2,0 - 15,0 mmol/g SiH bezogen auf das Polymer, besonders bevorzugt zwischen 2,0 - 9,5 mmol/g), mit einer Viskosität von 10 bis 10000 mPa·s, vorzugsweise von 30 bis 500 mPa·s, das zu-einem sehr hohen Haftverbund mit dem erfindungsgemäßen 1-K-Unterfütterungs-adhäsiv führt.

Darüberhinaus wird durch einen Zusatz von Organohydrogenpolysiloxanen a2) gemäß Anspruch 4 mit vergleichsweise geringen SiH-Gehalt (0,03 - 2,0 mmol/g SiH bezogen auf das Polymer), mit einer Viskosität von 10 bis 70000 mPa·s, vorzugsweise von 30 bis 2000 mPa·s, eine hohe Elastizität und Reißfestigkeit erreicht.

Die o.g. Organohydrogenpolysiloxane a1) können folgende Struktur aufweisen:

### A. Strukturformel der linearen Organohydrogensiloxane

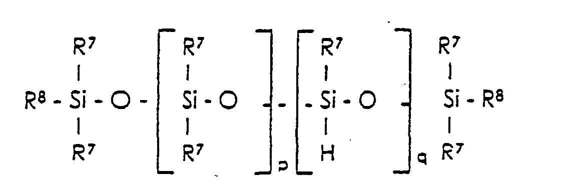

mit p = 0 bis 1500
mit q = 0 bis 1500
mit R⁷ = Alkyl- (z.B. Methyl-, Ethyl-, Isoproyl-), Aryl- (z.B. Phenyl-, Naphtyl-, Tolyl-, Xylyl-), Aralkyl- (Benzyl-, Phenylethyl-) und halogensubstituierte Alkyl- und Aryl-Gruppen (z.B. 3,3,3,-Trifluorpropyl-, Chlorphenyl-, Difluorphenyl-), Cyanalkyl-, Cycloalkyl- und Cycloalkenyl-. Vorzugsweise ist R = Methyl-.
mit R⁸ = R⁷, H
B. Si-H-haltige MQ-Harze bestehend aus:

(R⁷)₃SiO_{½}-, (R⁷)₂(H)SiO_{½}- und SiO_{4/2}-Einheiten.

Darüberhinaus können auch noch als T-Einheiten das trifunktionelle R⁸SiO_{3/2} und als D-Einheiten das bifunktionelle R⁷R⁸SiO_{2/2} vorhanden sein.
wobei R⁷ und R⁸ die unter A. genannte Bedeutung haben.

Die Gefahr des Gasens, d.h. des Abspaltens von H₂ aus den Organohydrogensiloxanen, wie sie durch Verunreinigungen und Restmengen aus dem Herstellungsprozeß vorhanden sein können, z.B. Reste von Säure, Basen und Metallen, wird durch die hohe Reinheit, den gewählten Konzentrationen bezogen auf die Gesamtrezeptur und durch den optimalen SiH-Gehalt minimiert bzw. ausgeschlossen.

Darüberhinaus können auch alle anderen Vorkehrungen getroffen werden, die nach dem Stand der Technik bekannt sind, um das Gasen zu vermeiden.

Die vinylhaltigen Organopolysiloxane b) mit zwei Vinylgruppen im Molekül zeigen folgende allgemeine Formel:

CH₂=CH-SiR₂O-(SiR₂O)ₙ-SiR₂-CH=CH₂,

worin
- R =: Alkyl- (z.B. Methyl-, Ethyl-, Isoproyl-), Aryl- (z.B. Phenyl-, Naphtyl-, Tolyl-, Xylyl-), Aralkyl- (Benzyl-, Phenylethyl-) und halogensubstituierte Alkyl- und Aryl-Gruppen (z.B. 3,3,3,-Trifluorpropyl-, Chlorphenyl-, Difluorphenyl-), Cyanalkyl-, Cycloalkyl- und Cycloalkenyl-. Vorzugsweise ist R = Methyl-.
- n =: eine ganze Zahl im Wert von 21 bis 1500,
wobei "n" theoretisch berechnete Werte darstellt, die aus dem experimentell bestimmten Vinylgehalt berechnet wurden, unter der Annahme, daß pro Molekül zwei Vinylgruppen enthalten sind und R = Methyl ist.

Als Katalysatoren c) für die Hydrosilylierung sind Salze, Komplexe und kolloidal vorliegende Formen der Übergangsmetalle der 8. Nebengruppe geeignet; vorzugsweise die Metalle Platin, Palladium und Rhodium; insbesondere erweisen sich Platinkomplexe, die z.B. aus Hexachloroplatinsäure bzw. aus Platinsalzen hergestellt werden, als geeignet. Diese Katalysatoren können auch auf Trägermaterial wie z.B. Zeolith oder Kohle aufgebracht sein.

Die Komponente d) ist ein hochdisperser, aktiver Füllstoff mit einer BET-Oberfläche von mindestens 25 m²/g wie Titandioxid, Aluminiumoxid, Zinkoxid und bevorzugterweise naßgefällte oder pyrogen gewonnene Kieselsäure.

Genannte Stoffe können hydrophil oder hydrophobiert vorliegen.

Weiterhin können als verstärkende Füllstoffe faser-oder blättchenförmige Füllstoffe eingesetzt werden, wobei mineralische, faserförmige Füllstoffe, wie z.B. Wollastonit und synthetische, faserförmige Füllstoffe, wie z.B. Glasfasern, Keramikfasern oder Kunststoffasern zum Einsatz kommen.

Verstärkende Füllstoffe werden in die Rezeptur eingearbeitet, um das Sedimentationsverhalten von ggf. enthaltenen nicht verstärkenden Füllstoffen zu verbessern und um die rheologischen Eigenschaften, wie Viskosität und Thixotropie einzustellen.

Füllstoffe e) werden in die Formulierungen eingearbeitet, um kostengünstigere Formulierungen zu erhalten und um innerhalb enger Grenzen, die mechanischen Eigenschaften zu beeinflussen.

Eingesetzt werden Metalloxide, Metallhydroxide, Mischoxide und Mischhydroxide, vorzugsweise Siliciumdioxid, insbesondere in Form von Quarz und Cristobalit sowie Aluminiumoxid, Calciumoxid, Aluminiumhydroxid.

Auch Füllstoffe wie Calciumcarbonat, Kieselgur, Diatommenerde, Talkum, Glas und Füllstoffe auf Kunststoffbasis, beispielsweise Polymethylmethacrylat-Perlpolymerisat, Polycarbonat, Polyvinylchlorid, Silicon-harzpulver, Pulver auf Basis fluororganischer Verbindungen sowie anorganische bzw. organische sphärische Füllstoffe und Fasern.

Die unter d) und e) genannten Füllstoffe können auch oberflächenbehandelt (gecoatet) vorliegen. Die Oberflächenbehandlung kann z.B. mit Silanen und Fettsäuren erfolgen, die funktionelle Gruppen aufweisen, die wiederum mit den reaktiven Komponenten a), b) und c) reagieren können, z.B.

H₂C = CH-(CH₂)ₙ - Si (OCH₃)₃ n = 0 bis 30

CH₂ = C(CH₃)- COO- (CH₂)ₙ - Si(OCH₃)₃.

Bei den unter f) genannten Farbstoffen handelt es sich um dieselben Verbindungen, wie sie beim Unterfütterungsadhäsiv unter f) beschrieben sind.

Als Feuchtigkeitsbinder g) können Zeolithe, wasserfreies Aluminiumsulfat, Molsieb, Kieselgur und Blaugel eingesetzt werden. Die Wirkung der Feuchtigkeitsbinder beruht auf der selektiven Adsorption von Wassermolekülen.

Bei der unter h) genannten Organopolysiloxanen mit mehr als zwei Vinylgruppen im Molekül handelt es sich um vinylendgestoppte und vinylseitenständige Organopolysiloxane mit einer Viskosität von 20 bis 350.000 mPa·s.

Als Inhibitoren i) zur Reaktivitäteinstellung der Hydrosilylierungsreaktion werden kurzkettige Organopolysiloxane der allgemeinen Formel

CH₂=CH-SiR₂O-(SiR₂O)ₙ-SiR₂-CH=CH₂

eingesetzt, worin
- R: gleiche oder verschiedene, ggf. substituierte Kohlenwasserstoffreste, wie z.B. Alkyl-, Alkenyl-, Alkinyl und
- n =: 0 oder eine ganze Zahl von 1 bis 6 bedeutet.
- R: kann weiterhin ein Alkenyl- oder Alkinyl-terminierter Siloxanrest sein.

Ferner können zum Regeln der Vernetzungsgeschwindigkeit vinylhaltige, cyclische Siloxane, wie beispielsweise Tetra-Vinyltetramethylcyclotetrasiloxan oder endständige Doppel- oder Dreifachbindungen enthaltende, organische Hydroxyl-Verbindungen eingesetzt werden.

Die unter j) genannten vinyl- und ethoxygruppenhaltigen, festen oder flüssigen MQ-Harze sind dadurch gekennzeichnet, daß sie als Q-Einheit das tetrafunktionelle SiO_{4/2} und als M-Bausteine das monofunktionelle R₃SiO_{1/2}, wobei R = Vinyl-, Methyl-, Ethyl-, Phenyl- sein kann, enthalten.

Darüber hinaus können auch noch als T-Einheiten das trifunktionelle RSiO_{3/2} und als D-Einheiten das bifunktionelle R₂SiO_{2/2} mit der gleichen Bedeutung für R wie oben vorhanden sein.

Diese MQ-Harze können in Organopolysiloxanen mit zwei oder mehr Vinylgruppen im Molekül und einer Viskosität von 21 bis 350.000 mPa·s gelöst vorliegen.

Der Vinylgruppengehalt der genannten MQ-Harze liegt im Bereich von 0,1 bis 8 mmol/g und der Ethoxygruppengehalt liegt bei kleiner 4 mmol/g. Der SiOH-Gehalt der MQ-Harze liegt so niedrig, daß kein Gasen durch Wasserstoffentwicklung auftritt.

Weiterhin ist der Anteil der flüchtigen Bestandteile der MQ-Harze so gering, daß die Dimensions-stabilität nicht beeinträchtigt wird.

Die Compounds k) setzen sich aus Organopolysiloxanen mit zwei oder mehr Vinylgruppen im Molekül und einer Viskosität von 21 bis 350.000 mPa·s und den unter d) genannten verstärkenden Füllstoffen zusammen. Diese können unter Verwendung von Modifizierhilfsmitteln, z.B. Hexamethyldisalazan in situ hydrophobiert werden.

Bei den als Tenside, Emulgatoren und Stabilisatoren eingesetzten Komponenten l) handelt es sich um dieselben Verbindungen, wie sie beim Unterfütterungsadhäsiv unter beschrieben sind.

Bei den unter m) genannten radioopaken Substanzen handelt es sich um barium-, strontium-, lanthan-oder zinkhaltige Gläser, Bariumsulfat, Zirkondioxid, Lanthanoxid oder um keramische Füllstoffzusammensetzungen, die Oxide von Lanthan, Hafnium und Seltenerdmetallen enthalten.

Weiterhin können komplexe Schwermetallfluoride der allgemeinen Formel M^{II}M^{IV}F₆ oder YF₃ eingesetzt werden, wobei M^{II} ein Calcium-, Strontium- oder Bariumion und M^{IV} ein Titan-, Zirkon-, oder Hafniumion bedeutet.

Ferner am Siliconpolymer gebundene Atome bzw. Atomgruppen, die radioopake Eigenschaften besitzen, wie z.B. an Silicium gebundenes Jod.

Bei den unter n) genannten H₂-Absorber/Adsorber handelt es sich um feinverteiltes Palladium oder Platin bzw. deren Legierungen, die ggf. in Alumosilikaten enthalten sind.

Weiterhin sind auch Substanzen einsetzbar, die die H₂-Entwicklung eliminieren bzw. reduzieren, wie z.B. 3-Methyl-1-butin-3-ol und CH₃Si[O-C(CH₃)₂-C ≡ CH]₃.

Bei den unter o) genannten Weichmachern, Neutral-und Verteilerölen handelt es sich um trimethylsiloxyendgestoppte Polydimethylsiloxane, Kohlenwasserstoffe, (Mineralöle, Wachse) Vaseline, Ester wie Adipinsäure-ester, Benzoesäureester, Buttersäureester, Essigsäureester, Ester höherer Fettsäuren, epoxidierte Fettsäureester, Glycolsäureester, höhermolekulare Ester (Polymerweichmacher), Phosphorsäureester, Propionsäureester, Sebacinsäureester, Sulfonsäureester, Trimellithsäureester, Citronensäureester, Abietinsäureester, Azelainsäureester, Ketone wie z. B. Campher, chlorierte Kohlenwasserstoffe, Polyole und Polyolether.

Der erfindungsgemäße Unterfütterungs-Versiegelungslack besteht aus zwei Komponenten, die über eine edelmetallkatalysierte Hydrosilylierungsreaktion chemisch vernetzen.

Die Komponenten A und B des Unterfütterungs-Versiegelungslacks können in verschiedenen Mischungsverhältnissen eingestellt werden. In der Regel werden die beiden Komponenten im Mischungsverhältnis 1:1 gewichts- oder volumenmäßig über geeignete Dosier- und Mischvorrichtungen wie Stranglängendosierung und manuelles Mischen, Kartuschensysteme mit statischem Mischer oder elektrisch/mechanisch betriebene Mischgeräte gemischt.

Die Viskositäten der Komponenten A und B sowie die Viskosität der homogenen Mischung von A und B zum Zeitpunkt nach Ende des Mischens werden vorzugsweise auf 25 bis 10000 mPa·s eingestellt. Auf diese Weise ist gewährleistet, daß der Unterfütterungs-Versiegelungslack während seiner Verarbeitungszeit in dünnster Schicht mit geeigneten Hilfsmitteln, wie Pinsel, Schwamm oder Spatel, auf die zu versiegelnden Bereiche der unterfütterten Prothese auftragbar ist.

Bei der Herstellung einer Unterfütterung ist es zur exakten Anpassung des im Mund ausgehärteten Unterfütterungssiliconmaterials an die individuellen Mund- und Kieferkonturen erforderlich, das Material mit einer Fräse oder einem Skalpell zu bearbeiten.

Die durch diese mechanische Bearbeitung "verletzten" angerauhten Flächen des Unterfütterungssilicon und der Übergangsbereich von Unterfütterungsadhäsiv zu Unterfütterungssilicon können mit dem erfindungsgemäßen Unterfütterungs-Versiegelungslack durch Aufbringen einer dünnen Schicht der homogen gemischten Paste während der Gesamtverarbeitungszeit versiegelt werden.

Auf diese Weise werden Porositäten und Rauhigkeiten vermieden, die während der Tragedauer der unterfütterten Prothese die Anlagerung von Plaque, Speiseresten, Bakterien und Pilzen begünstigen könnten. Durch Vermeidung von Rauhigkeiten wird gleichzeitig der Tragekomfort der unterfütterten Prothese erhöht, da die versiegelten Bereiche die Reibung zwischen unterfütterter Prothese und der Schleimhaut reduzieren. Somit wird die bei Unterfütterungs-Patienten häufig durch Schleimhautentzündung, scharfe Knochenkanten, frische Extraktionen oder Operationsgebiete geschädigte Mundschleimhaut zusätzlich entlastet und sogenannte "Radiergummieffekte" vermieden.

Der erfindungsgemäße Unterfütterungs-Versiegelungslack zeichnet sich gegenüber dem Stand der Technik durch eine besonders hohe Reißfestigkeit und seine glatte Oberflächenbeschaffenheit aus.

Überraschenderweise wurde festgestellt, daß es möglich ist, hohe Reißfestigkeiten trotz niedrigster Viskositäten des Unterfütterungs-Versiegelungslacks zu erzielen. Die Reißfestigkeiten von Unter-fütterungs-Versiegelungslacken nach dem Stand der Technik erweisen sich als nicht in der Lage, mögliche Dehn-, Stauch- oder Biegebewegungen des zugrundeliegenden Unterfütterungssilicons unbeschadet zu überstehen. Aufgrund deren hoher Brüchigkeit kann es nach der mechanischen Belastung zur Rißbildung in der Unterfütterungs-Versiegelungslackschicht kommen, was die Unterfütterung wieder anfällig gegen Anlagerung von Verunreinigungen macht.

Der erfindungsgemäße Unterfütterungs-Versiegelungs-lack erfüllt die geforderten Reißfestigkeiten und Anforderungen an den Haftverbund zu den Funktionseinheiten Unterfütterungssilicon und Unterfütterungsadhäsiv unter Beibehaltung der sonstigen mechanischen und verarbeitungsfreundlichen Eigenschaften hinsichtlich sinnvoll verarbeitbarer Viskositäten mit den oben genannten Misch- und Dosiersystemen bzw. Auftrags-hilfsmitteln, wie Pinsel oder Schwamm, praxisorientiertem Abbindeverhalten bei Raumtemperatur, Fließverhalten während der Gesamtverarbeitungszeit und glatter Oberflächenbeschaffenheit.

Weiterhin zeichnet sich der Unterfütterungs-Versiegelungslack neben einer praxisgerechten Verarbeitbarkeit und einem guten Fließverhalten während der Verarbeitungszeit durch ein anwenderfreundliches Abbinden auf der zu versiegelnden Fläche bei Raumtemperatur aus.

Durch die beschriebenen Leistungsmerkmale des Unter-fütterungssiliconlacks wird der Tragekomfort und die Paßgenauigkeit der unterfütterten Prothese weiter verbessert und führt zu einer hohen Akzeptanz beim Patienten.

Überraschenderweise wurde festgestellt, daß durch Kombination von Organohydrogenpolysiloxanen mit hohem SiH-Gehalt (2,0 bis 15 mmol/g) bezogen auf das Polymer und besonders bevorzugt zwischen 3,0 und 11 mmol/ g, mit Organohydrogenpolysiloxane gemäß Anspruch 9 mit mit geringem SiH-Gehalt (0,05 bis 2 mmol/g) eine besonders hohe Elastizität und Reißfestigkeit im Vergleich zum Stand der Technik erreicht werden kann.

Durch Verwendung entsprechend kurzkettiger Organopolysiloxane mit zwei Vinylgruppen im Molekül können die für eine dünne Auftragbarkeit der Unterfütterungs-siliconlackschicht erforderlichen Viskositäten von 25 bis 10000 mPa·s erzielt werden.

Die im Unterfütterungs-Versiegelungslack beinhalteten Organohydrogenpolysiloxane gewährleisten eine hohe Haftkraft sowohl zum Unterfütterungssilicon als auch zum Unterfütterungsadhäsiv.

Die Formulierungen des Unterfütterungs-Versiegelungslacks verfügen über eine ausreichende Lagerstabilität in der entsprechenden Primärverpackung (z.B. Tube oder Kartusche). Unter Lagerstabilität in diesem Zusammenhang ist die Aufrechterhaltung der Leistungsmerkmale vor und während der Vulkanisation hinsichtlich der Viskositäten der Einzelkomponenten A und B und der Mischung, sowie der Abbindecharakteristik über mindestens 12 Monate und nach der Vulkanisation hinsichtlich der Reißfestigkeit und Haftkraft über mindestens 12 Monate zu verstehen.

Weiterhin zeigt der Unterfütterungs-Versiegelungslack nach Wasserlagerung und nach Kochen in Wasser keinen Abfall der Reißfestigkeit. Auch der Haftverbund zum Unterfütterungssilicon und zum Unterfütterungsadhäsiv bleibt nach oben genannter Behandlung erhalten (siehe Tabelle 4).

Folgende Zusammensetzung von Inhaltsstoffen und folgende Mengen der einzelnen Komponenten a) bis n) in Gew.%, bezogen auf das gesamte Siliconmaterial (Komponenten A und B) wird erfindungsgemäß verwendet:
a1) 0,1 bis 80 Gew.%, vorzugsweise 1 bis 40 Gew.% Organohydrogenpolysiloxane und deren Mischungen und einem SiH-Gehalt von 2,0 - 15 mmol/g, vorzugsweise 3,0 - 11,0 mmol/g, mit einer Viskosität von 10 bis 10000 mPa·s, vorzugsweise von 30 bis 500 mPa·s
a2) 0,1 bis 80 Gew.%, vorzugsweise 1 bis 40 Gew.% Organohydrogenpolysiloxane gemäß Anspruch 9 und deren Mischungen und einem SiH-Gehalt von 0,05 - 2,0 mmol/g, mit einer Viskosität von 10 bis 10000 mPa·s, vorzugsweise von 30 bis 2000 mPa·s
b) 1 bis 90 Gew.%, vorzugsweise 10 bis 60 Gew.% Organopolysiloxane mit zwei Vinylgruppen im Molekül und einer Viskosität von 21 bis 200.000 mPa·s
c) 0,0001 bis 1,0 Gew.% vorzugsweise 0,0005 bis 0,2 Gew.% der Katalysatoren zur Beschleunigung der Hydrosilylierungsreaktion, bezogen auf reines Metall
d) 0 bis 80 Gew.%, vorzugsweise 0 bis 50 Gew.% der verstärkenden Füllstoffe
e) 0 bis 90 Gew.%, vorzugsweise 20 bis 80 Gew.% der nicht verstärkenden Füllstoffe
f) 0 bis 10 Gew.%, vorzugsweise 0 bis 2 Gew.% der Farbstoffe
g) 0 bis 50 Gew.%, vorzugsweise 0 bis 10 Gew.% der Feuchtigkeitsbinder
h) 0 bis 70 Gew.%, vorzugsweise 0 bis 20 Gew.% der Organopolysiloxane mit mehr als zwei Vinylgruppen pro Molekül
i) 0 bis 1,0 Gew.%, vorzugsweise 0 bis 0,1 Gew.% der Inhibitoren
j) 0 bis 90 Gew.%, vorzugswesie 0 bis 50 Gew.% des vinylgruppenhaltigen festen oder flüssigen MQ-Harzes
k) 0 bis 80 Gew.%, vorzugsweise 0 bis 50 Gew.% eines Compounds aus vinylgruppenhaltigen Organopolysiloxanen und verstärkenden Füllstoffen
l) 0 bis 10 Gew.%, vorzugsweise 0 bis 5 Gew.% der Tenside, Emulgatoren und Stabilisatoren
m) 0 bis 90 Gew.%, vorzugsweise 0 bis 80 Gew.% der radioopaken Substanzen
n) 0 bis 20 Gew.%, vorzugsweise 0 bis 10 Gew.% der H₂-Absorber/Adsorber oder der die H₂-Ent-wicklung reduzierenden bzw. eliminierenden Substanzen.

Die Inhaltsstoffe des Unterfütterungslacks entsprechen den Inhaltsstoffen der Unterfütterungs-silicons bzw.
Unterfütterungsadhäsivs.

Zur erfindungsgemäßen Herstellung der Unterfütterung werden zunächst die zu klebenden Bereiche des PMMA-Prothesenkunststoffs gereinigt und/oder aufgerauht, beispielsweise durch Korundstrahlen mit Korund einer Partikelgröße von ca. 100 µm bei einem Druck von 2 bar.

Auf diese Bereiche wird das erfindungsgemäße Ein Komponenten-Adhäsiv mit einer Schichtdicke von 0,1 bis 500 µm, vorzugsweise 0,1 bis 20 µm aufgebracht. Das erfindungsgemäße Adhäsiv kann dabei in flüssiger oder gelöster Form auf den zu klebenden PMMA-Prothesenkunststoff aufgebracht werden. Nach Abdampfen des Lösungsmittels kann das erfindungsgemäße Unterfütterungssilicon sofort aufgebracht werden.

Zur Beschleunigung der Bildung der Haftvermittlerschicht (z.B. bei Verwendung höher siedender Lösungsmittel) bzw. zur Erhöhung des Haftverbundes der Haftvermittlerschicht zwischen Silicon und PMMA-Prothesenkunststoff kann auch eine Temperung bei Temperaturen > 30°C durchgeführt werden.

Bei der direkten Unterfütterungsmethode (chairside) wird die mit Unterfütterungsadhäsiv und Unterfütterungssilicon versehene Prothese in den Patientenmund eingebracht.

Während der Verarbeitungszeit des Unterfütterungs-silicons wird eine Funktionsabformung vorgenommen, d.h. die mimischen Bewegungen des Patienten werden vom Unterfütterungssilicon aufgezeichnet. Nach Entnahme der ausgehärteten Unterfütterung kann diese durch Schneiden oder Fräsen zügig weiter bearbeitet werden.

Die durch das Bearbeiten entstandene Oberfächenrauhigkeiten werden mit dem erfindungsgemäßen Unterfütterungs-Versiegelungslack in einer Schichtdicke von 0,1 bis 1000 µm, vorzugsweise 0,1 bis 200 µm versiegelt.

Weitere vorteilhafte Ausgestaltungen der Erfindung sind in den Ansprüchen gekennzeichnet.

Nachstehend wird die Erfindung anhand von Beispielen näher erläutert.

### 1. Herstellung eines Unterfütterungsadhäsivs

Zu 1,5 Teilen Dibenzoylperoxid werden 400 Teile Methylmethacrylat und 100 Teile α-Methacryloxy-propyl-ω-vinyltetramethyldisiloxan zugegeben. Die Mischung wird bei Raumtemperatur gerührt bis sich das Dibenzoyxiperoxid gelöst hat. Danach werden im Argon-Strom 1,5 Teile N,N-Dimethyl-toluidin zugegeben. Die erhaltene Reaktionslösung wird unter Argon-Schutzgas unter Rühren auf 40°C erwärmt. Nach beendeter Reaktionszeit, z.B. nach 40 min. erhält man ein schwach gelblich gefärbtes Produkt.

### 2. Herstellung eines Unterfütterungsadhäsivs als Lösung

5 Teile des unter Beispiel 1 hergestellten Produktes werden in 95 Teilen Dichlormethan gelöst. Die erhaltene Lösung stellt eine Ausführungsform des erfindungsgemäßen 1-Komponenten-Unterfütterungsadhäsivs dar.

### 3. Herstellung eines weichen Unterfütterungssilicons

### a) Herstellung der Komponente A

In einem geschlossenen Kneter werden 82 Teile eines vinylendgestoppten Polydimethylsiloxans mit einer Viskosität von 10.000 mPa·s bei 20°C mit 17 Teilen einer pyrogen hergestellten, hochdispersen, hydrophobierten Kieselsäure mit einer Oberfläche von 200 m²/g nach BET, 1 Teil eines in Polydimethylsiloxan gelösten Platin-Katalysators mit einem Gehalt an reinem Platin von 1 % für 1,5 Stunden homogenisiert und danach 15 Minuten im Vakuum entgast.

Man erhält eine mittelfließende Paste. Die Paste stellt eine mögliche Ausführungsform der Komponente A des erfindungsgemäßen weichen Unterfütterungssilicons dar.

Nach Lagerung bei 23°C über einen Monat waren die Viskosität und die Reaktivität der Paste unverändert im Sollwertbereich.

### b) Herstellung der Komponente B

In einem geschlossenen Kneter werden 68 Teile eines vinylendgestoppten Polydimethylsiloxans mit einer Viskosität von 10.000 mPa.s bei 20°C mit 7 Teilen einer 1:1-Mischung zweier Polydimethylhydrogensiloxane mit SiH-Gehalten von 0,33 bzw. 4,3 mmol/g, 25 Teilen einer pyrogen hergestellten, hochdispersen, hydrophobierten Kieselsäure mit einer Oberfläche von 200m²/g nach BET für 1,5 Stunden homogenisiert und danach 15 Minuten im Vakuum entgast.

Man enthält eine mittelfließende Paste (DIN EN 24823). Die Paste stellt eine mögliche Ausführungsform der Komponente B des erfindungsgemäßen Unterfütterungssilicons dar.

Nach Lagerung bei 23°C über einen Monat waren die Viskosität und die Reaktivität der Paste unverändert im Sollwertbereich.

### c) Mischung der Komponenten A und B des Unterfütterungs-silicons

50 Teile der unter 3a) beschriebenen Komponente A und 50 Teile der unter 3b) beschriebenen Komponente B werden aus einer Kartusche ausgedrückt und über einen statischen Mischer homogen gemischt. Das Produkt bleibt eine Minute bei Raumtemperatur und zusätzlich zwei Minuten bei einer Temperatur von 35°C verarbeitbar und bindet innerhalb von zehn Minuten nach Mischbeginn vollständig ab.

Man erhält als Vulkanisat weiche, hochelastische Formkörper, die nach 1 Woche im Zugversuch (DIN 53455) Reißfestigkeiten von 502 N/cm² bei einer Dehnung von 718% erreichen.

Die mechanische Bearbeitbarkeit des entstandenen Vulkanisats ist im Gegensatz zu den Handelsprodukten 2 und 3 mit einer rotierenden Fräse (Typ: Meisinger HM72SX, Drehzahl: 25000 U/min.) gut möglich. Selbst in dünnen Schichten lassen sich feine Strukturen herausfräsen, ohne daß ein Radiergummieffekt auftritt oder größere Siliconbruchstücke unkontrolliert herausgerissen werden.

### 4. Herstellung eines weichen Unterfütterungssilicons mit extrem hoher Reißfestigkeit

### a) Herstellung der Komponente A

In einem geschlossenen Kneter werden 69 Teile eines vinylendgestoppten Polydimethylsiloxans mit einer Viskosität von 1000 mPa·s bei 20°C mit 30 Teilen einer pyrogen hergestellten, hochdispersen, hydrophobierten Kieselsäure mit einer Oberfläche von 200 m²/g nach BET, 1 Teil eines in Polydimethylsiloxan gelösten Platin-Katalysators mit einem Gehalt an reinem Platin von 1 % für 1,5 Stunden homogenisiert und danach 15 Minuten im Vakuum entgast.

Man erhält eine mittelfließende Paste. Die Paste stellt eine mögliche Ausführungsform der Komponente A des erfindungsgemäßen weichen Unterfütterungssilicons dar.

Nach Lagerung bei 23°C über einen Monat waren die Viskosität und die Reaktivität der Paste unverändert im Sollwertbereich.

### b) Herstellung der Komponente B

In einem geschlossenen Kneter werden 50 Teile eines vinylendgestoppten Polydimethylsiloxans mit einer Viskosität von 1000 mPa·s bei 20°C mit 6 Teilen eines vinylendgestoppten Polydimethylsiloxans mit einer Viskosität von 10000 mPa·s bei 20°C, 32 Teilen einer pyrogen hergestellten, hochdispersen, hydrophobierten Kieselsäure mit einer Oberfläche von 200 m²/g nach BET, 1 Teil eines Polydimethylsiloxans mit end- und seitenständigen Vinylgruppen mit einer Viskosität von 5000 mPa·s bei 20°C und einem Vinylgehalt von 0,8 mmol/g, 1 Teil eines MQ-Festsiliconharzes mit einem Vinylgehalt von 0,7 mmol/g, mit 10 Teilen einer 1:1-Mischung zweier Polydimethylhydrogensiloxane mit SiH-Gehalten von 0,13 bzw. 4,3 mmol/g für 1,5 Stunden homogenisiert und danach 15 Minuten im Vakuum entgast.

Man erhält eine mittelfließende Paste. Die Paste stellt eine mögliche Ausführungsform der Komponente B des erfindungsgemäßen Unterfütterungssilicons dar.

Nach Lagerung bei 23°C über einen Monat waren die Viskosität und die Reaktivität der Paste unverändert im Sollwertbereich.

### c) Mischung der Komponenten A und B des Unterfütterungssilicons

50 Teile der unter 4a) beschriebenen Komponente A und 50 Teile der unter 4b) beschriebenen Komponente B werden aus einer Kartusche ausgedrückt und über einen statischen Mischer homogen gemischt.

Das Produkt bleibt eine Minute bei Raumtemperatur und zusätzlich eine Minute bei einer Temperatur von 35°C verarbeitbar und bindet innerhalb zehn Minuten nach Mischbeginn vollständig ab.

Man erhält als Vulkanisat weiche, hochreißfeste Formkörper, die nach 24 Stunden im Zugversuch (DIN 53455) Reißfestigkeiten von 670 N/cm² bei einer Dehnung von 120% erreichen.

Die mechanische Bearbeitbarkeit ist analog zu Beispiel 3c).

### 5. Herstellung eines harten Unterfütterungssilicons

### a) Herstellung der Komponente A

In einem geschlossenen Kneter werden 650 Teile eines vinylendgestoppten Polydimethylsiloxans mit einer Viskosität von 200 mPa·s bei 20°C mit 340 Teilen einer pyrogen hergestellten, hochdispersen, hydrophobierten Kieselsäure mit einer Oberfläche von 200 m²/g nach BET und 10 Teilen eines in Polydimethylsiloxans gelösten Platin-Katalysators mit einem Gehalt an reinem Platin von 1 % für 1,5 Stunden homogenisiert und danach 15 Minuten im Vakuum entgast.

Man erhält eine mittelfließende Paste, die eine mögliche Ausführungsform der Komponente A des erfindungsgemäßen, harten Unterfütterungssilicons darstellt.

Nach Lagerung bei 23°C über einen Monat waren die Viskosität und die Reaktivität der Paste unverändert im Sollwertbereich.

### b) Herstellung der Komponente B

In einem geschlossenen Kneter werden 400 Teile eines vinylendgestoppten Polydimethylsiloxans mit einer Viskosität von 200 mPa·s bei 20°C mit 400 Teilen einer Mischung zweier Polydimethylhydrogensiloxane mit SiH-Gehalten von 0,33 bzw. 6,9 mmol/g, 300 Teilen einem pyrogen hergestellten, hochdispersen, hydrophobierten Kieselsäure mit einer Oberfläche von 200 m²/g nach BET für 1,5 Stunden homogenisiert und danach 15 Minuten im Vakuum entgast. Man erhält eine mittelfließende Paste (DIN EN 24823). Die Paste stellt eine mögliche Ausführungsform der Komponente B des erfindungsgemäßen Unterfütterungs-silicons dar.

Nach Lagerung bei 23°C über einen Monat waren die Viskosität und die Reaktivität der Paste unverändert im Sollwertbereich.

### c) Mischung der Komponenten A und B des Unter-fütterungssilicons

50 Teile der unter 5a) beschriebenen Komponente A und 50 Teile der unter 5b) beschriebenen Komponente B werden aus einer Kartusche ausgedrückt und über einen statischen Mischer homogen gemischt.

Das Produkt bleibt eine Minute bei Raumtemperatur und zusätzlich zwei Minuten bei einer Temperatur von 35°C verarbeitbar und bindet innerhalb von zehn Minuten nach Mischbeginn vollständig ab.

Man erhält als Vulkanisat harte, zähelastische Formkörper, die nach 1 Woche im Zugversuch (DIN 53455) Reißfestigkeiten von 533 N/cm² bei einer Dehnung von 77% erreichen.

Die mechanische Bearbeitbarkeit ist analog zu Beispiel 3c).

### 6. Herstellen eines Standard-Dentalsilicons mit mittelfließender Konsistenz

### a) Herstellung der Komponente A

In einem geschlossenen Kneter werden 20 Teile eines vinylendgestoppten Polydimethylsiloxans mit einer Viskosität von 1000 mPa·s und 20 Teile eines vinylendgestoppten Polydimethylsiloxans mit einer Viskosität von 10000 mPa·s mit 3 Teilen einer pyrogen hergestellten, hoch-dispersen, hydrophobierten Kieselsäure mit einer Oberfläche von 200 m²/g nach BET, 56 Teilen eines Quarzfeinmehls und 1 Teil eines in Polydimethylsiloxan gelösten Platinkatalysators mit einem Gehalt an reinem Platin von 1% für 1,5 Stunden homogenisiert und danach 15 Minuten im Vakuum entgast.

Man erhält eine mittelfließende Paste, die die Komponente A einer Standard-Dentalmasse darstellt.

### b) Herstellung der Komponente B

In einem geschlossenen Kneter werden 20 Teile eines vinylendgestoppten Polydimethylsiloxans mit einer Viskosität von 10000 mPa·s und 7 Teile eines vinylendgestoppten Polydimethylsiloxans mit einer Viskosität von 65000 mPa·s mit 6 Teilen eines Organohydrogenpolysiloxans mit einem SiH-Gehalt von 2,3 mmol/g und einer Viskosität von 230 mPa·s, und 3 Teilen einer pyrogen hergestellten, hochdispersen, hydrophobierten Kieselsäure mit einer Oberfläche von 200 m²/g nach BET und 55 Teilen eines Quarzfeinmehls für 1,5 Stunden homogenisiert und danach 15 Minuten im Vakuum entgast.

Man erhält eine mittelfließende Paste, die die Komponente B einer Standard-Dentalmasse darstellt.

### c) Mischung der Komponenten A und B des Standard-Dentalsilicons

50 Teile der unter 6a) beschriebenen Komponente A und 50 Teile der unter 6b) beschriebenen Komponente B werden mit Hilfe eines Mischspatels auf dem Mischblock homogen gemischt. Das Produkt hat eine Verarbeitungszeit von 1 min. 30 sec. und bindet innerhalb von 6 min. nach Mischbeginn ab.

Man erhält als Vulkanisat einen mittelharten, mäßig reißfesten Formkörper, der nach 1 Woche RT-Lagerung im Zugversuch (DIN 53455) Reißfestigkeiten von 200 N/cm² bei einer Drehung von 190% erreicht.

### 7. Herstellung des Unterfütterungs-Versiegelungslacks

### a) Komponente A

In einem Laborplanetendissolver werden 60 Teile eines vinylendgestoppten Polydimethylsiloxans mit einer Viskosität von 200 mPa·s bei 20°C, 15 Teilen eines in Organopolysiloxan gelösten MQ-Festsiliconharzes mit 1,0 mmol/ g Vinylgehalt und einer Viskosität von 2000 mPa·s bei 20°C, mit 15 Teilen einer pyrogen hergestellten, hochdispersen, hydrophobierten Kieselsäure mit einer Oberfäche von 200 m²/g nach BET und einem Teil eines Trimethylsiloxyendgestoppten Polydimethylsiloxans mit einer Viskosität von 50 mPa·s bei 20°C sowie 0,5 Teilen eines in Polydimethylsiloxan gelösten Platin-Katalysators mit einem Gehalt an reinem Platin von 1 % für 1,5 Stunden homogenisiert und danach 15 Minuten im Vakuum entgast.

### b) Komponente B

In einem Laborplanetendissolver werden 35 Teile eines vinylendgestoppten Polydimethylsiloxans mit einer Viskosität von 200 mPa·s bei 20°C, 20 Teile einer Lösung eines MQ-Harzes in α-, ω-Divinylpolydimethylsiloxan mit 1,0 mmol/g Vinylgehalt und einer Viskosität von 2000 mPa.s bei 20°C, ein Teil eines trimethylsiloxyendgestoppten Polydimethylsiloxan mit einer Viskosität von 50 mPa·s bei 20°C, 16 Teile einer pyrogen hergestellten, hochdispersen, hydrophobierten Kieselsäure mit einer Oberfläche von 200 m²/g nach BET, 18 Teile eines Organohydrogenpolydimethylsiloxans mit einem SiH-Gehalt von 7 mmol/g und 15 Teile eines Organohydrogenpolydimethylsiloxans mit einem SiH-Gehalt von 0,33 mmol/g für 1,5 Stunden homogenisiert und danach 15 Minuten im Vakuum entgast.

Man erhält eine sehr dünnfließende Paste mit einer Viskosität von 3000 mPa·s, die eine mögliche Ausführungsform der Komponente B des erfindungsgemäßen Unterfütterungssilicon-Versiegelungslacks darstellt.

Nach Lagerung bei 23°C über einen Monat waren die Viskosität und die Reaktivität der Paste unverändert im Sollwertbereich.

### c) Mischung der Komponenten A und B des Unter-fütterungssilicon-Versiegelungslacks

50 Teile der unter 7a) beschriebenen Komponente A und 50 Teile der unter 7b) beschriebenen Komponente B werden aus einer Kartusche ausgedrückt und über einen statischen Mischer homogen gemischt.

Das Produkt bleibt bei Raumtemperatur zwei Minuten bearbeitbar, d.h. es läßt sich zwei Minuten mit einem Pinsel verstreichen, und bindet innerhalb von 8 Minuten nach Mischbeginn bei Raumtemperatur vollständig ab.

Man erhält als Vulkanisat weiche, elastische Formkörper, die nach 48 Stunden im Zugversuch (DIN 53455) Reißfestigkeiten von 115 N/cm² bei einer Dehnung von 144% erreichen.

### 8. Haftverbund PMMA - efindungsgemäßes Adhäsiv-erfindungsgemäßes Silicon

Aus einem kalthärtenden Prothesenkunststoff (Kaltpolymerisat, Heißpolymerisat und Spritzgußprothesenkunststoff) werden gemäß Herstellerangaben zwei Prüfkörper der Abmessungen 40 mm x 12,5 mm x 5 mm (Länge x Breite x Dicke) angefertigt, mit Schleifpapier (Körnung 240) angeschliffen und durch Anblasen mit Druckluft gereinigt.

Das Adhäsiv aus Punkt 2 und zum Vergleich das Adhäsiv von Handelsprodukt 2 wird mit einem Pinsel dünn auf die beiden gereinigten PMMA-Prüfkörper aufgetragen und eine Minute liegen-gelassen, damit das Lösungsmittel vollständig abdampfen kann.

Danach wird das gemäß Punkt 3c) angemischten Unterfütterungssilicon und zum Vergleich das angemischte Unterfütterungssilicon von Handelsprodukt 2 auf die mit Adhäsiv aus Punkt 2 bzw. dem Adhäsiv von Handelsprodukt 2 bestrichene Seite eines PMMA-Prüfkörpers aufgebracht. Der zweite PMMA-Prüfkörper wird mit der mit Adhäsiv bestrichenen Seite so auf das Silicon auf dem ersten PMMA-Prüfkörper gelegt, daß eine quadratische Überlappungsfläche von 1 cm² beider Prüfkörper entsteht.

Die Anordnung wird auf der Überlappungsfläche für 15 Sekunden mit 500 g Gewicht belastet und danach 15 Minuten bei 35°C im Wasserbad und anschließend 16 Stunden bei Raumtemperatur gelagert.

Der Haftverbund der Prüfkörperanordnung wird im Zugversuch ermittelt.

Die mechanische Bearbeitung des auf dem Prothesenkunststoff haftenden Unterfütterungssilicon mit einer rotierenden Fräse (Typ: Meisinger HM 72 SX, Drehzahl 25000 U/min.) ist gut möglich, auch bei dünnen Schichten. Darüberhinaus ist bei dieser mechanischen Bearbeitung kein Ablösen des Unterfütterungssilicon vom Prothesenkunststoff zu beobachten.

Wie die Werte aus Tabelle 2b) zeigen, weist das erfindungsgemäße Unterfütterungssystem (Bsp. aus Punkt 2 und 3c) im Vergleich zu Handelsprodukt 2 bei allen 3 Prothesenkunststoffen die höheren Haftverbunde auf.

### 9. Prüfung der Verbundfestigkeit zwischen Prothesenbasismaterial und Unterfütterungssilicon nach Dauerwechselbelastung.

Die Dauerwechselbelastungsprüfung simuliert die während der Tragedauer der Prothese auftretenden Kräfte, z.3. Kaubelastungen.

Ein Prothesenträger wendet nach Haraldson, Karlsson und Carlsson (J Oral Rehab 6, 41-48 (1979)) an "komfortabler Stelle" eine maximale Kraft von 77 N auf. Wenn man dies mit 100 N nach oben abschätzt (entsprechend etwa dem Dreifachen der normalen Kaukraft) und bei einseitiger Prothesenbelastung eine druckaufnehmende Fläche von 300 mm² annimmt, ergibt sich eine Druckspannung von etwa 0,3 N/mm², der die Prothese bzw. ein Unterfütterungsmaterial maximal ausgesetzt wäre. Diese entspricht bei einem "weichen" Silikon (E-Modul etwa 1 N/mm²) einer Stauchung εvon 30%, die während des Wechselbelstungstests realisiert werden müßte, Um die in praxi vorkommende Scherbelastung mit zu erfassen, wird am Prüfkörper gleichzeitig eine Scherung γ derselben Größenordnung angelegt. Wie in der Realität sollte es sich um Schwellbelastungen handeln (zwischen null und einem Maximum pendelnd) mit einer Druckspannungsamplitude von 0,3 N/mm² und demzufolge ca. 0,1 N/mm² Scherspannungsamplitude (entsprechend ε=0,3 und γ =0,3)

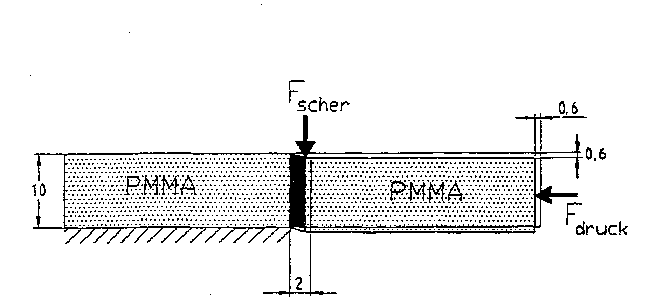

Fig. 1 verdeutlich die geometrischen Veränderungen des Prüfkörpers während der Schwellbelastung, wobei der weichbleibende, dunkel schraffierte Kunststoff zwischen zwei PMMA-Blöcken unter kombinierter Druck- und Scherbelastung liegt. Die Maße sind dabei in mm angegeben.

Die Tests werden gleichzeitig an drei Prüfkörpern durchgeführt, um mit geringsmöglichen zeitlichem Aufwand eine repräsentative Stichprobe beurteilen zu können. Simulation des Mundmilieus ist durch Wasserberieselung (37 °C) realisiert. 10⁶ Lastwechsel entsprechend 500 bis 1000 Tagen klinischer Funktionsdauer. Die Qualität der Verbindung PMMA/ weichbleibender Kunststoff wird mit der abgebideten Prüfkörpervariante am elegantesten und am empfindlichsten im Zugtest ermittelt und im direkten Vergleich zu bei 37°C, 48 Std. im Wasserbad gelagerten Prüfkörpern beurteilt.

Wie die Ergebnisse aus Tabelle 2c zeigen, tritt bei der Verbundfestigkeit zwischen dem erfindungsgemäßen Unterfütterungssilicon (Beispiel 4.3.c) und dem erfindungsgemäßen Unterfütterungsadhäsiv (Beispiel 2) im Vergleich zu den Handelsprodukten kein Abfall des Haftverbundes nach 10⁶ Lastwechseln auf.

Tabelle 3 zeigt die/den Reißfestigkeit/Haftverbund des erfindungsgemäßen Unterfütterungssystems bei verschiedenen Lagerbedingungen. Wie die Werte belegen, ist kein nennenswerter Abfall der/des Reißfestigkeit/Haftverbundes zu beobachten.

**Tabelle 1**

| Vergleichende Untersuchungen zu Unterfüllerungssiliconen | | | | | | | |
|---|---|---|---|---|---|---|---|
| | Reißfestigkeit (N/cm²)¹⁾ | | | Dehnung(%)¹⁾ | | | Handkraft zum Austragen (N)²⁾ |
| | 1 Wo. RT | 2 Wo. 60°C | 12 h 100°C | 1 Wo. RT | 1 Wo. 60°C | 12 h 100°C | |
| Beispiel 4.3 c) | 502 | 553 | 556 | 718 | 628 | 582 | 332 |
| Beispiel 4.4 c) | 670 | 680 | 680 | 120 | 110 | 105 | 380 |
| Beispiel 4.5 c) | 530 | 446 | 482 | 77 | 54 | 39 | 353 |
| Beispiel 4.6 c) | 200 | - | - | 190 | - | - | - |
| Handelsprodukt 1 (Charge: 587) | 138 | 177 | 202 | 351 | 327 | 365 | 757 |
| Handelsprodukt 2 (Charge: 72123) | 250 | 242 | 300 | 711 | 545 | 586 | 730 |
| Handelsprodukt 3 (Charge: 01) | 210 | 146 | 171 | 651 | 420 | 402 | 396 |

| | | | | | | | |
|---|---|---|---|---|---|---|---|
| ¹⁾ Die Reißfestigkeiten und Dehnungen werden an einer Universalprüfmaschine der Firma Zwick Z010 in Anlehnung an DIN 53455 gemessen (S2-Schulterstäbe, Prüfgeschwindigkeit 50 mm/min.) ²⁾ Die Handkraft zum Austragen wird an einer gefüllten 48 ml-Kartusche mit aufgesetztem statischen Mischer im Druckversuch (Prüfgeschwindigkeit 50 mm/min.) an einer Universalprüfmaschine der Firma Zwick Z010 mit einer speziellen Kartuschenhalterung ermittelt. ³⁾ Handelsprodukt 1: Tokuyama Soft Relining von Tokuyama Corp., Tokyo Handelsprodukt 2: Ufi-Gel C von Firma VOCO, Cuxhafen Handelsprodukt 3: Mollosil Plus von Firma Detax, Karlsruhe | | | | | | | |

**Tabelle 2 a)**

| Vergleichende Untersuchungen zum Haftverbund zwischen Unterfütterungssiliconen und Unterfütterungsadhäsiv | | | | | | |
|---|---|---|---|---|---|---|
| | 15 min./35 °C | | 1 Woche/60 °C | | 12 Std./100 °C Wasserbad | |
| | Haftverbund Bruchspannung N/cm² | Bruchbild | Haftverbund Bruchspannung N/cm² | Bruchbild | Haftverbund Bruchspannung N/cm² | Bruchbild |
| Beispiel 4.3 c) | 179 | 100 % | 284 | 100 % | 412 | 100 % |
| Handelsprodukt 1 (Charge: 587) | 75 | 100 % | 67 | 100 % | 114 | 100 % |
| Handelsprodukt 2 (Charge: 72123) | 114 | 60 % | 109 | 100 % | 141 | 70 % |
| Handelsprodukt 3 (Charge: 01) | 101 | 80 % | 132 | 100 % | 233 | 90 % |

**Tabelle 2 b)**

| Vergleichende Untersuchungen zum Haftverbund zwischen Unterfütterungssiliconen und Unterfütterungsadhäsiv mit verschiedenen Prothesenkunststoffen (Messung nach 16 Std. Lagerung bei Raumtemperatur) | | | | | | |
|---|---|---|---|---|---|---|
| | Kaltpolymerisat | | Heißpolymerisat | | Spritzguß-Prothesenkunsttoff | |
| | Haftverbund Bruchspannung N/cm² | Bruchbild [%] | Haftverbund Bruchspannung N/cm² | Bruchbild [%] | Haftverbund Bruchspannung N/cm² | Bruchbild[%] |
| Beispiel 4.3 c) | 186 | 100 | 216 | 100 | 144 | 80 |
| Handelsprodukt 2 (Charge: 72123) | 103 | 100 | 99 | 50 | 111 | 40 |

| | | | | | | |
|---|---|---|---|---|---|---|
| ¹ = Paladur, Kulzer ² = Paladon 65, Kulzer ³ = SR Ivocap plus, Ivoclar | | | | | | |

**Tabelle 2c**

| Verbundfestigkeit zwischen Prothesenbasismaterial und Unterfütterungssilicon nach Deuerwechselbelastung: | | |
|---|---|---|
| | 48 Std./37°C Ausgangswert vor Wechselbelastung | nach 10⁶ Wechselbelastungen |
| | [MPa] | [MPa] |
| Beispiel 4.3.c) | 2,0 | 2,1 |
| Handelsprodukt 1 | 0,9 | 0,8 |
| Handelsprodukt 2 | 1,8 | 1,5 |
| Handelsprodukt 3 | 1,7 | 1,2 |

**Tabelle 3**

| Stress-Test am Unterfütterungssystem aus den Beispielen 4.1 c) und 4.3 c) | | | | |
|---|---|---|---|---|
| Lagerung | Dauer [Wochen] | Bruchspannung [N/cm²]Verbundprüfkörper | Bruchbild [%] | Bruchspannung S2 Schulterstab [N/cm²] |
| Luft ²⁾ | 5 | 224 | 100 | 483 |
| | | | | |
| Wasser ³⁾ | 5 | 200 | 100 | 463 |
| | | | | |
| Speichel ⁴⁾ | 6 | 229 | 100 | 529 |
| | | | | |
| Kaffee ⁵⁾ | 6 | 243 | 100 | 473 |
| | | | | |
| Wasser. Zigarettenrauchgesättigt ⁶⁾ | 5 | 171 | 100 | 584 |
| | | | | |
| Fernet Brance ⁷⁾ | 6 | 211 | 100 | 468 |
| | | | | |
| Weinbrand ⁸⁾ | 6 | 194 | 100 | 434 |
| | | | | |
| Reinigungsmittel Helon⁹⁾ | 5 | 239 | 100 | 493 |
| 2-Phasen ¹⁰⁾ | 5 | 221 | 100 | 494 |
| Corega Raucher ¹¹⁾ | 5 | 217 | 100 | 515 |
| Express ¹²⁾ | 5 | 216 | 100 | 508 |
| Corega Bio¹³⁾ | 5 | 200 | 100 | 528 |
| Mundspülung Corega ¹⁴⁾ | 5 | 196 | 100 | 506 |
| | | | | |
| Desinfektionsmittel Perform ¹⁵⁾ | 4 | 329 | 100 | 579 |
| Stamm Z ¹⁶⁾ | 4 | 320 | 100 | 532 |
| | | | | |
| Desinfektionsmittel Lyso P ¹⁷⁾ | 4 | 301 | 100 | 532 |
| Lyso d ¹⁸⁾ | 4 | 274 | 100 | 565 |
| Stamm D ¹⁹⁾ | 4 | 281 | 100 | 521 |
| | | | | |
| Temperaturwechsel ²⁰⁾ | 5 | 206 | 100 | 551 |

| | | | | |
|---|---|---|---|---|
| ¹⁾ Die Stress-Tests wurden jeweils an drei Prüfkörpern pro Versuchsreihe durchgeführt, die gemäß dem Verfahren nach Beispiel 4.8 hergestellt wurden. Die Bruchspannung wurde im Zugversuch an einer Universalprüfmaschine der Firma Zwick Z010 gemessen (Prüfgeschwindigkeit 10 mm/min.). Das Bruchbild wird visuell im Anschluß an die Bestimmung der Bruchspannung beurteilt. Ein Bruchbild von 100 % bedeutet den Idealfall eines rein kohäsiven Bruchs im Silicon. Beide Seiten der PMMA-Prüfkörper sind nach dem Bruch 100 % mit Silicon bedeckt. Der Haftverbund zwischen PMMA und Silicon ist unberührt und vollständig erhalten. Ein Bruchteil von 0 % entspricht einem adhäsiven Bruch zwischen PMMA und Silicon. Das Silicon ist unberührt. Bruchbilder zwischen 0 und 100 % bedeuten entsprechende Abstufungen in der Beurteilung. ²⁾ Normklima 23 °C und 50 % Luftfeuchtigkeit gemäß DIN 24823. ³⁾ Das Wasser wurde mit 0,1 % Lebensmittelfarbstoff eingefärbt. Eine Einfärbung des Unterfütterungssilicons und des Klebebereichs konnte nicht beobachtet werden. ⁴⁾ 10 % Hydroxyethylstärke (HES 200/0,5) in isotonischer NaCl-Lösung der Firma Fresenius, Ch.-Nr. GC 1011, unverdünnte Flüssigkeit. ⁵⁾ Eduscho Business Daytime, Dosierung: 60 g auf 1 I Wasser, gebrüht bei 100 °C. ⁶⁾ Wasser, gesättigt mit Zigarettenrauch (Firma Marlboro), Ch.-Nr. 03111. ⁷⁾ Gold Krone der Firma Nordbrand Nordhausen GmbH, Ch.-Nr. L340609, unverdünnte Flüssigkeit. ⁸⁾ Fernet Branca der Firma Fratelli Branca, Ch.-Nr. L5891, unverdünnte Flüssigkeit. ⁹⁾ Helon Zahnprothesenreiniger der Firma Dental Kosmetik GmbH High Dent, Dresden, Ch.-Nr. 17063141, Dosierung: 3 g auf 150 ml Wasser (2 %ige Lösung), Lösung tägl. Erneuert. ¹⁰⁾ Blend a dent 2-Phasen-Reiniger der Firma Wick Pharma, Schwalbach, Ch.-Nr. 62368, Dosierung: 1 Tablette auf 150 ml Wasser, Lösung tägl. erneuert. ¹¹⁾ Corega Raucher Tabs der Firma Block Drug Company Inc., Ratingen, Ch.-Nr. 6FO3A 1, Dosierung: 1 Tablette auf 150 ml Wasser, Lösung tägl. erneuert. ¹²⁾ Blend a dent Express Reinigungsgranulat der Firma Wick Pharma, Schwalbach, Ch.-Nr. 6145, Dosierung: 1 Portion auf 150 ml Wasser, Lösung tägl. erneuert. ¹³⁾ Corega Tabs Bio Formel der Firma Block Drug Company Inc., Ratingen, Ch.-Nr. T6132, Dosierung: 1 Tablette pro 150 ml Wasser, Lösung tägl. erneuert. ¹⁴⁾ Corega Mundspülung der Firma Block Company Inc., Ratingen, Ch.-Nr. 94627, unverdünnte Flüssigkeit. ¹⁵⁾ Perform der Firma Kettenbach, Eschenburg. Dosierung: 40 g auf 2 l Wasser (2 %ig), Lösung tägl. erneuert. ¹⁶⁾ Stammopur Z der Firma Stamm, Berlin, Ch.-Nr. 961211, Dosierung: 5 %ige Lösung in Wasser, Lösung tägl. erneuert. ¹⁷⁾ Lysoform P der Dr. H. Rosemann GmbH. Ch.-Nr. 101225, Dosierung: 6 %ige Lösung in Wasser, Lösung tägl. erneuert. ¹⁸⁾ Lysoform D der Dr. H. Rosemann GmbH, Ch.-Nr. 021306, Dosierung: 2,5 %ige Lösung in Wasser, Lösung tägl. erneuert. ¹⁹⁾ Stammoform D der Firma Stamm. Berlin, Ch.-Nr. 1041269, Dosierung: 10 g auf 500 ml Wasser (2 %ig). Lösung tägl. erneuert. ²⁰⁾ Innerhalb von 5 Wochen wurden die in Wasser gelagerten Prüfkörper Temperaturwechseln von + 5 °C auf + 60 °C bzw. + 60 °C auf + 5 °C ausgesetzt. Es konnte keine Beeinträchtigung der Bruchspannung und des Bruchbildes festgestellt werden. | | | | |

**Tabelle 4**

| Vergleichende Untersuchungen zu Unterfütterungssilicon-Versiegelungslacken | | | | |
|---|---|---|---|---|
| | Reißfestigkeit | Dehnung | Haftverbung zum Silicon | Haftverbund zum Adhäsiv |
| Beispiel 4.6 c) | 115 | 144 | 100 % | 100 % |
| | | | | |
| Handelsprodukt 1 (Charge: 91121 P) | 18 | 80 | 2) | 2) |
| | | | | |
| Handelsprodukt 2 (Charge: 72091) | 2) | 2) | 2) | 2) |
| | | | | |
| Handelsprodukt 3 (Charge: 970301/401 | 2) | 2) | 2) | 2) |

Reißfestigkeit und Dehnung werden in Anlehnung an DIN 53504 nach 2 Tagen an S 2 Schulterstäben gemessen.

Die mechanischen Eigenschaften von Handelsprodukt 2 und Handelsprodukt 3 sowie teilweise Handelsprodukt 1 sind so unzureichend, daß die technischen Daten nicht ermittelbar sind.

## Patentansprüche

1. Unterfütterungsadhäsiv mit folgender Zusammensetzung:
a) Adhäsivkomponente Z als Reinsubstanz 0 bis 99 Gew.-%,
b) Copolymer mit der Adhäsivkomponente Z als Bestandteil 0 bis 99 Gew.-%,
c) Copolymer in der Mischung mit den Synthese-Ausgangsstoffen aus der Polymerisation (Adhäsivkomponente Z, Monomer) 0 bis 99 Gew.-%
wobei die Summe der Gewichtsanteile der Bestandteile a), b) und c) mindestens 0,01 Gew.-% ist, und wobei sich die Mischung c) des Copolymers mit den Synthese-Ausgangsstoffen aus der Polymerisation, Adhäsivkomponente Z und Monomer, aus folgenden Bestandteilen zusammensetzt:
- dem Copolymer 0,1 bis 99,9 Gew. %,
- der Adhäsivkomponente Z 0,1 bis 99,9 Gew. %
- dem Monomer 0,1 bis 99 Gew.-%
wobei die Summe der drei Bestandteile 100% ergibt,
d) Katalysatoren zur Beschleunigung der Ausbildung des Haftverbundes zum zu verklebenden Silicon 0 bis 10 Gew.-%,
e) Lösungsmittel 1 bis 99,99 Gew. %,
f) Farbstoffe 0 bis 20 Gew. %,
g) Tenside, Emulgatoren und Stabilisatoren 0 bis 20 Gew.-%,
h) Organohydrogensiloxan 0 bis 40 Gew.-%,
i) α, ω-Divinylpolydialkylsiloxan 0 bis 40 Gew. %,
wobei,
Z folgende allgemeine Formel aufweist: 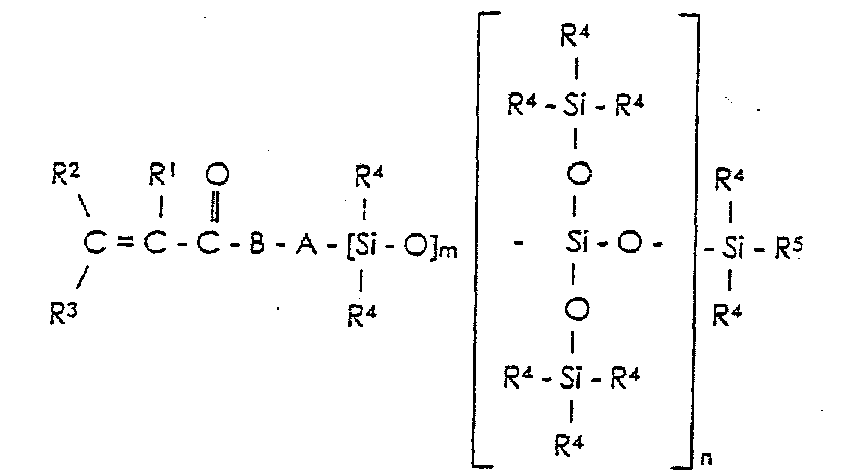 wobei n und m ganze Zahlen im Bereich von Null bis 1.000 sind,
R¹: H, Halogen, Alkyl, substituiertes Alkyl, CN,
R², R³: H, Halogen, Alkyl, substituiertes Alkyl und
B = O, S, NH, NR, PH, PR,
A= Spacer, geradkettiger oder verzweigter Kohlenwasserstoffrest mit 0 bis 30-C Atomen, der auch einen aromatisch ungesättigten oder cycloaliphatischen Ring enthalten kann,
R⁴ eine ungesättigte Gruppe und/oder eine der folgenden Gruppen: Alkyl-, Aryl-, Aralkyl- und halogensubstituierte Alkyl- und Aryl-Gruppen, Cyanalkyl, Cycloalkyl,
darstellt und
R⁵ gleich R⁴ oder R⁶ sein kann, wobei R⁶ sich zusammensetzt aus den Struktureinheiten, nämlich
A, B, Polydialkylsiloxy-Einheit und der ethylenisch ungesättigten Einheit:

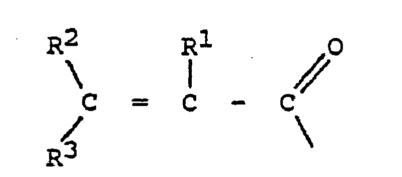

2. Unterfütterungsadhäsiv nach Anspruch 1, **dadurch gekennzeichnet, daß** als Monomere Methacrylate und Acrylate, vorzugsweise mono- oder polyfunktionelle Methacrylate, insbesondere Alkylmethacrylat, Cycloalkylmethacrylat, lsoalkyl-methacrylat, Tetraethylenglycoldimethacrylat, Triethylenglycoldimethacrylat, Diethylenglycoldimethacrylat, Ethylenglykoldimethacrylat, Polyethylenglykol-dimethacrylat, Butandioldimethacrylat, Hexandioldimethacrylat, Decandioldi-methacrylat, Dodecandioldimethacrylat, Bisphenol-A-dimethacrylat, Trimethylol-propantrimethacrylat, 2, 2-Bis-4 (3-methacryloxy-2-hydroxy)-phenylpropan (Bis GMA) sowie Reaktionsprodukte aus Di- und/oder Triisocyanaten und OH-gruppenhaltigen Methacrylaten verwendet werden.

3. Unterfütterungsadhäsiv nach Anspruch 1 oder 2, **dadurch gekennzeichnet, daß** als Lösungsmittel organische Lösungsmittel wie Aromate, Benzine, Ether, Ester, Alkohole, Ketone, Aldehyde, Amine, Carbonsäuren, halogenierte Lösungsmittel, z.B. Chloroform und Wasser sowie die möglichen Kombinationen aus oben genannten Lösungsmitteln eingesetzt werden.

4. 2-Komponenten-Unterfütterungssilicon mit folgender Zusammensetzung
a1) 0,1 - 80 Gew.-% Organohydrogenpolysiloxane oder deren Mischungen mit einem SiH-Gehalt von 2,0 - 15,0 mmol/g SiH bezogen auf das Polymer, mit einer Viskosität von 10 bis 10.000 mPas,
a2) 0,1 - 80 Gew.-% Organohydrogenpolysiloxane oder deren Mischungen mit einen SiH-Gehalt von 0,03 - 2,0 mmol/g SiH bezogen auf das Polymer, mit einer Viskosität von 10 bis 70.000 mPas,
b) 1 bis 90 Gew.-% Organosiloxane mit zwei Vinylgruppen im Molekül,
c) 0,0001 bis 1,0 Gew.-% Katalysatoren, bezogen auf den reinen Edelmetallgehalt,
d) 0 bis 80 Gew.-% verstärkende Füllstoffe,
e) 0 bis 90 Gew.-% nicht verstärkende Füllstoffe,
f) 0 bis 10 Gew.-% Farbstoffe,
g) 0 bis 50 Gew.-% Feuchtigkeitsbinder,
h) 0 bis 70 Gew.-% Organopolysiloxane, mit mehr als zwei Vinylgruppen im Molekül
i) 0 bis 1,0 Gew.-% Inhibitoren,
j) 0 bis 90 Gew.-% festes oder -flüssiges MQ-Siliconharz,
k) 0 bis 80 Gew.-% Compound,
l) 0 bis 10 Gew.-% Tenside, Emulgatoren, Stabilisatoren,
m) 0 bis 90 Gew.-% radioopake Substanzen,
n) 0 bis 20 Gew.-% H₂-Absorber/Adsorber oder die H₂-Entwicklung reduzierende oder eliminierende Substanzen,
o) 0 bis 50 Gew.-% Weichmacher, Neutralöle, Verteileröle, wobei die Organohydrogenpolysiloxane a2) folgende Struktur aufweisen:
A. Strukturformel der linearen Organohydrogensiloxane 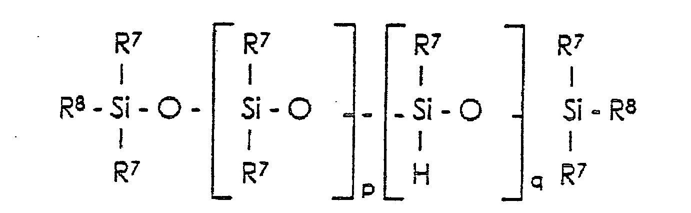 mit p = 0 bis 1.500
mit q = 1 bis 1.500
mit R⁷ = Alkyl-, Aryl-, Aralkyl- und halogensubstituierte Alkyl- und Aryl-Gruppen, Cyanalkyl, Cycloalkyl und Cycloalkenyl
mit R⁸ = H.

5. 2-Komponenten-Unterfütterungssilicon gemäß Anspruch 4, wobei die Organohydrogenpolysiloxane a1) folgende Struktur aufweisen:
A. Strukturformel der linearen Organohydrogensiloxane 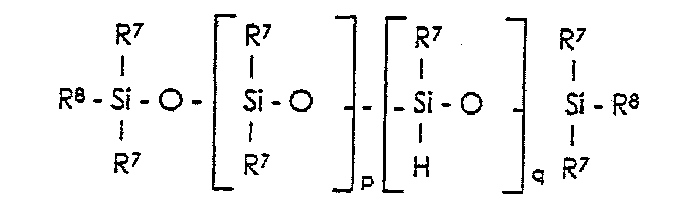 mit p = 0 bis 1.500
mit q = 0 bis 1.500
mit R⁷ = Alkyl-, Aryl-, Aralkyl- und halogensubstituierte Alkyl- und Aryl-Gruppen, Cyanalkyl, Cycloalkyl und Cycloalkenyl
mit R⁸ = R⁷, H,
B. SiH-haltige MQ-Harze bestehend aus:
(R⁷)₃SiO_{1/2}, (R⁷)₂(H)SiO_{1/2} und SiO_{4/2}-Einheiten, wobei auch noch als T-Einheiten das trifunktionelle R⁸SiO_{3/2} und als D-Einheiten das bifunktionelle R⁷R⁸SiO_{2/2} vorhanden sein können,
wobei R⁷ und R⁸ die unter A. genannten Bedeutungen haben.

6. 2-Komponenten-Unterfütterungssilicon nach Anspruch 4 oder 5, **dadurch gekennzeichnet, daß** die vinylhaltigen Organopolysiloxane mit zwei Vinylgruppen im Molekül die allgemeine Formel:
CH₂=CH-SiR₂O-(SiR₂O)ₙ-SiR₂-CH=CH₂
aufweisen, worin
R = Alkyl-, Aralkyl- und halogensubstituierte Alkyl- und Aryl-Gruppen, Cyanalkyl, Cycloalkyl und Cycloalkenyl und
n = eine ganze Zahl im Wert von 21 bis 1.500, wobei "n" theoretisch berechnete Werte darstellt, die aus dem experimentell bestimmten Vinylgehalt berechnet wurden, unter der Annahme, daß pro Molekül zwei Vinylgruppen enthalten sind und R = Methyl ist.

7. 2-Komponenten-Unterfütterungssilicon nach einem der Ansprüche 4 bis 6, **dadurch gekennzeichnet, daß** der Katalysator ein Salz, Komplex oder eine kolloidal vorliegende Form der Übergangsmetalle der 8. Nebengruppe darstellt.

8. 2-Komponenten-Unterfültterungssilicon nach einem der Ansprüche 4 bis 7, **dadurch gekennzeichnet, daß** die Füllstoffe d) des Unterfütterungssilicons hydrophobiert oder hydrophil sind und/oder daß die Füllstoffe d) und/oder e) des Unterfütterungssilicons oberflächenbehandelt sind, wobei Silane und/oder Fettsäuren verwendet wurden, die funktionelle Gruppen aufweisen, die mit den Komponenten a), b) und c) reagieren können.

9. 2-Komponenten-Versiegelungslack, wobei die beiden Komponenten über eine edelmetallkatalysierte Hydrosilylierungsreaktion chemisch vernetzen, bei denen es sich um ein Organohydrogenpolysiloxan mit hohem SiH Gehalt von 2,0 bis 15 mmol/g bezogen auf das Polymer und ein Organohydrogenpolysiloxan mit einem geringen SiH-Gehalt von 0,05 bis 2 mmol/g bezogen auf das Polymer handelt, wobei der Versiegelungslack eine Viskosität von 25 bis 10,000 mPas aufweist, wobei der 2-Komponenten-Versiegelungslack die gleichen Inhaltsstoffe aufweist wie das 2-Komponenten-Unterfütterungssilicon gemäß einem der Ansprüche 4 bis 8, die in den gleichen prozentualen Anteilen vorliegen können.

10. 2-Komponenten-Versiegelungslack nach Ansprach 9, **dadurch gekennzeichnet, daß** der 2-Komponenten-Versiegelungslack in einer Kartusche vorliegt.

11. Verfahren zur Herstellung einer direkten Unterfütterung, die nach einem der Ansprüche 1 bis 7 aufgebaut ist, **dadurch gekennzeichnet, daß**
- der Prothesenkunststoff, der mit einer Unterfütterung versehen werden soll, gereinigt und/oder aufgerauht wird,
- anschließend das 1-Komponenten-Adhäsiv gemäß einem der Anspräche 1 bis 3 in einer Schichtdicke von 0,1 bis 500 µm aufgebracht wird,
- gegebenenfalls anschließend bei > 30°C für mindestens 5 Minuten getempert wird,
- nach Abdampfung des Lösungsmittels das 2-Komponenten Unterfütterungs-silicon gemäß einem der Ansprüche 4 bis 8 aufgebracht wird,
- während der Verarbeitungszeit des Unterfütterungssilicons eine Funktions-abformung vorgenommen wird,
- nach dem Aushärten des Unterfütterungssilicons eine Bearbeitung durch Schneiden oder Fräsen erfolgt und, daß die durch die Bearbeitung entstandenen Oberflächenrauhigkeiten mit dem Versiegelungslack gemäß Anspruch 9 in einer Schichtdicke von 0,1 bis 1.000 µm versiegelt werden.

12. Verfahren zur Herstellung einer indirekten Unterfütterung, die nach einem der Ansprüche 1 bis 7 aufgebaut ist, **dadurch gekennzeichnet, daß**
- der Prothesenkunststoff der mit einer Unterfütterung versehen werden soll gereinigt und/oder aufgerauht wird,
- anschließend das 1-Komponenten-Adhäsiv gemäß mindestens einem der Ansprüche 1 bis 3 in einer Schichtdicke von 0,1 bis 500 µm aufgebracht wird,
- gegebenenfalls anschließend bei > 30°C für mindestens 5 Minuten getempert wird,
- nach Abdampfung des Lösungsmittels das 2-Komponenten Unterfütterungs-silicon gemäß mindestens einem der Ansprüche 4 bis 8 aufgebracht wird,
- nach dem Aushärten des Unterfütterungssilicons eine Bearbeitung durch Schneiden oder Fräsen erfolgt
- die durch die Bearbeitung entstandenen Oberflächenrauhigkeiten mit dem Versiegelungslack gemäß Anspruch 9 in einer Schichtdicke von 0,1 bis 1,000 µm versiegelt werden.

13. Verfahren nach einem der Ansprüche 11 oder 12, **dadurch gekennzeichnet, daß** das Adhäsiv gemäß mindestens einem der Ansprüche 1 bis 3 in flüssiger oder gelöster Form verwendet wird.

14. Unterfütterungssystem, **dadurch gekennzeichnet, daß** es ein Unterfütterungs-adhäsiv gemäß mindestens einem der Ansprüche 1 bis 3, ein 2 Komponenten-Unterfütterungssilicon gemäß mindestens einem der Ansprüche 4 bis 8 und ein Versiegelungslack gemäß mindestens einem der Ansprüche 9 oder 10 enthält.

15. Unterfütterung, **dadurch gekennzeichnet, daß** sie aus einem Unterfütterungs-adhäsiv gemäß mindestens einem der Ansprüche 1 bis 3, einem 2-Komponenten-Unterfütterungssilicon gemäß mindestens einem der Ansprüche 4 bis 8 und einem Versiegelungslack gemäß mindestens einem der Ansprüche 9 oder 10 hergestellt ist.

## Claims

1. A relining adhesive having the following composition:
a) an adhesive component Z as a pure substance from 0 to 99% by weight;
b) a copolymer having the adhesive component Z as a component from 0 to 99% by weight;
c) a copolymer in admixture with the starting materials of the polymerization (adhesive component Z, monomer) from 0 to 99% by weight;
wherein the sum of the parts by weight of components a), b), and c) is at least 0.01% by weight and the mixture c) of the copolymer with the starting materials of the polymerization, the adhesive component Z and the monomer, is composed of the following components:
- the copolymer from 0.1 to 99.9% by weight,
- the adhesive component Z from 0.1 to 99.9% by weight,
- the monomer from 0.1 to 99% by weight,
the sum of all three components being 100%;
d) catalysts for accelerating the formation of the adhesive bond to the silicone to be bonded from 0 to 10% by weight;
e) a solvent from 1 to 99.99% by weight;
f) colourants from 0 to 20% by weight;
g) surfactants, emulsifiers, and stabilisers from 0 to 20% by weight;
h) an organohydrogensiloxane from 0 to 40% by weight;
i) an α,ω-divinylpolydialkylsiloxane from 0 to 40% by weight;
wherein
Z has the following general formula: 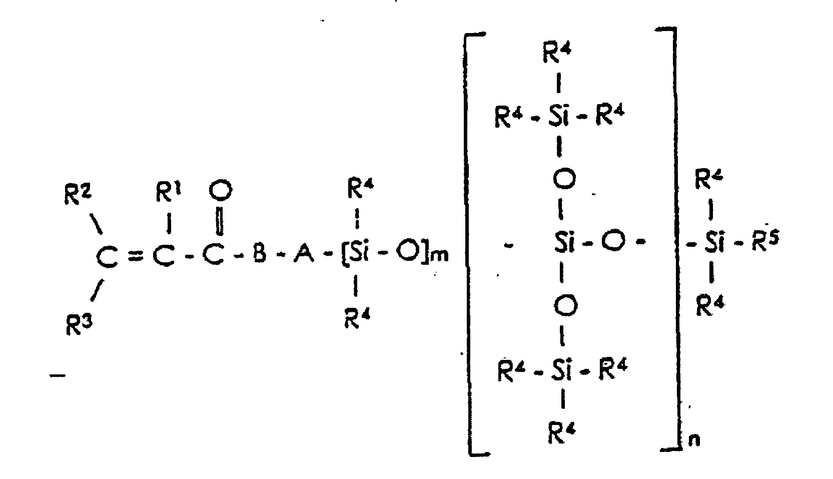 wherein n and m are integers in the range from 0 to 1000;
R¹: H, halogen, alkyl, substituted alkyl, CN;
R², R³: H, halogen, alkyl, substituted alkyl and
B = O, S, NH, NR, PH PR;
A = a spacer or a straight-chain or branched hydrocarbon moiety having from 0 to 30 C atoms which may also contain an aromatically unsaturated or a cycloaliphatic ring;
R⁴ is an unsaturated group and/or one of the following groups: alkyl, aryl, aralkyl, and halogenosubstituted alkyl and aryl groups, cyanoalkyl, cycloalkyl and
R⁵ may be equal to R⁴ or R⁶; R⁶ being composed of the moieties, namely
A, B, the polydialkylsiloxy unit and the ethylenically unsaturated unit:

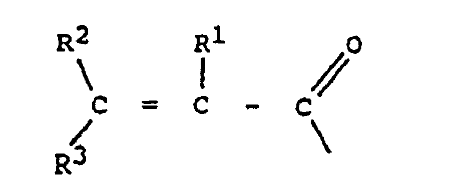

2. The relining adhesive according to claim 1, **characterized in that** methacrylates and acrylates, preferably mono- or polyfunctional methacrylates, in particular alkyl methacrylate, cycloalkyl methacrylate, isoalkyl methacrylate, tetraethylene glycol dimethacrylate, triethylene glycol dimethacrylate, diethylene glycol dimethacrylate, ethylene glycol dimethacrylate, polyethylene glycol dimethacrylate, butanediol dimethacrylate, hexanediol dimethacrylate, decanediol dimethacrylate, dodecanediol dimethacrylate, bisphenol A dimethacrylate, trimethylolpropane trimethacrylate, 2,2-bis-4(3-methacryloxy-2-hydroxy)phenylpropane (bis-GMA) and reaction products from di- and/or triisocyanates and OH group-containing methacrylates are used as monomers.

3. The relining adhesive according to claim 1 or 2, **characterized in that** organic solvents such as aromatics, petroleum spirits, ethers, esters, alcohols, ketones, aldehydes, amines, carboxylic acids, halogenated solvents, e.g. chloroform, and water and the possible combinations of the above-mentioned solvents can be used as solvents.

4. A two-component relining silicone having the following composition
a1) from 0.1 to 80% by weight of organohydrogenpolysiloxanes or mixtures thereof having a SiH content from 2.0 to 15.0 mmol/g SiH, based on the polymer, and a viscosity from 10 to 10,000 mPa·s,
a2) from 0.1 to 80% by weight of organohydrogenpolysiloxanes or mixtures thereof having a SiH content from 0.03 - 2.0 mmol/g SiH, based on the polymer, and a viscosity from 10 to 70,000 mPa·s,
b) from 1 to 90% by weight of organosiloxanes containing two vinyl groups within the molecule;
c) from 0.0001 to 1.0% by weight of catalysts, based on the content of the pure noble metal;
d) from 0 to 80% by weight of reinforcing fillers; e) from 0 to 90% by weight of non-reinforcing fillers;
f) from 0 to 10% by weight of colourants;
g) from 0 to 50% by weight of moisture binders;
h) from 0 to 70% by weight of organopolysiloxanes containing more than two vinyl groups within the molecule;
i) from 0 to 1.0% by weight of inhibitors;
j) from 0 to 90% by weight of solid or liquid MQ silicone resin;
k) from 0 to 80% by weight of the compound;
l) from 0 to 10% by weight of surfactants, emulsifiers, stabilisers;
m) from 0 to 90% by weight of radiopaque substances;
n) from 0 to 20% by weight of H₂ absorbers/adsorbers or substances reducing or eliminating the development of H₂;
o) from 0 to 50% by weight of plasticizers, neutral oils, distributing oils, wherein the organohydrogenpolysiloxanes a2) have the following structure:
A. structural formula of the linear organo-hydrogensiloxanes 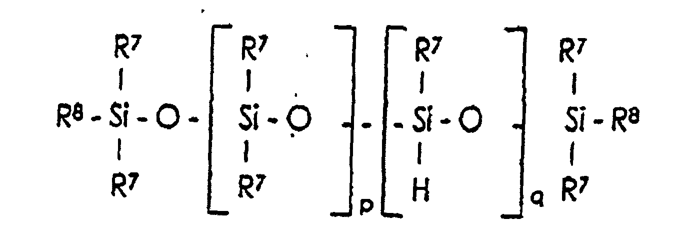 with p = 0 to 1500;
with q = 0 to 1500;
with R⁷ = alkyl, aryl, aralkyl, and halogenosubstituted alkyl and aryl groups, cyanoalkyl, cycloalkyl and cycloalkenyl;
with R⁸ = H.

5. The two-component relining silicone according to claim 4, wherein the organohydrogenpolysiloxanes a1) have the following structure:
A. structural formula of the linear organohydrogensiloxanes 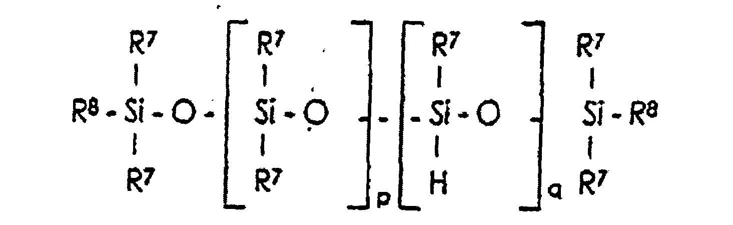 with p = 0 to 1500;
with q = 0 to 1500;
with R⁷ = alkyl, aryl, aralkyl, and halogenosubstituted alkyl and aryl groups, cyanoalkyl, cycloalkyl and cycloalkenyl;
with R⁸ = R⁷, H,
B. SiH-containing MQ resins consisting of:
(R⁷)₃SiO_{1/2}, (R⁷)₂(H)SiO_{1/2} and SiO_{4/2} units, where additionally the trifunctional R⁸SiO_{3/2} can be present as T units and the bifunctional R⁷R⁸SiO_{2/2} can be present as D units,
wherein R⁷ and R⁸ have the meanings defined in A.

6. The two-component relining silicone according to claim 4 or 5, **characterized in that** the vinyl-containing organopolysiloxanes having two vinyl groups within the molecule have the general formula:
CH₂=CH-SiR₂O-(SiR₂O)ₙ-SiR₂-CH=CH₂
wherein
R = alkyl, aralkyl, and halogenosubstituted alkyl and aryl groups, cyanoalkyl, cycloalkyl, and cycloalkenyl and
n = an integer having a value from 2 to 1500, "n" representing theoretically calculated values calculated from the experimentally determined vinyl content on the assumption that each molecule contains two vinyl groups and R = methyl.

7. The two-component relining silicone according to any one of claims 4 to 6, **characterized in that** the catalyst is a salt, a complex, or a colloidal form of the transition metals of the 8th subgroup of the periodic table.

8. The two-component relining silicone according to any one of claims 4 to 7, **characterized in that** the fillers d) of the relining silicone are hydrophobicized or hydrophilic and/or the fillers d) and/or e) of the relining silicone are surface-treated, wherein silanes and/or fatty acids containing functional groups which can react with components a), b), and c) are used.

9. A two-component sealing lacquer, wherein both components chemically crosslink by a hydrosilylation reaction catalysed by noble metals, said components being an organohydrogenpolysiloxane having a high SiH content from 2.0 to 15 mmol/g, based on the polymer, and an organohydrogenpolysiloxane having a low SiH content from 0.05 to 2 mmol/g, based on the polymer, the sealing lacquer having a viscosity from 25 to 10,000 mPa·s, wherein the two-component sealing lacquer contains the same ingredients as the two-component relining silicone according to any one of claims 4 to 8 and said ingredients can be present in the same percentages.

10. The two-component sealing lacquer according to claim 9, **characterized in that** the two-component sealing lacquer is contained within a cartridge.

11. A process for producing a direct relining constructed according to any one of claims 1 to 7, **characterized in that**
- the prosthesis plastic to be provided with a relining is cleaned and/or roughened;
- subsequently the one-component adhesive according to any one of claims 1 to 3 is applied in a layer thickness from 0.1 to 500 µm;
- optionally it is subsequently annealed at > 30°C for at least 5 min;
- after evaporation of the solvent the two-component relining silicone according to any one of claims 4 to 8 is applied;
- a functional impression is performed during the pot life of the relining silicone;
- after the curing of the relining silicone a processing by cutting or milling is performed and the surface roughnesses resulting from the processing are sealed by the sealing lacquer according to claim 9 in a layer thickness from 0.1 to 1000 µm.

12. A process for producing an indirect relining constructed according to any one of claims 1 to 7, **characterized in that**
- the prosthesis plastic to be provided with a relining is cleaned and/or roughened;
- subsequently the one-component adhesive according to at least one of claims 1 to 3 is applied in a layer thickness from 0.1 to 500 µm;
- optionally it is subsequently annealed at > 30°C for at least 5 min;
- after evaporation of the solvent the two-component relining silicone according to at least one of claims 4 to 8 is applied;
- after the curing of the relining silicone a processing by cutting or milling is performed;
- the surface roughnesses resulting from the processing are sealed by the sealing lacquer according to claim 9 in a layer thickness from 0.1 to 1000 µm.

13. The process according to any one of claims 11 or 12, **characterized in that** the adhesive according to at least one of claims 1 to 3 is used in a liquid or dissolved form.

14. A relining system, **characterized in that** it includes a relining adhesive according to at least one of claims 1 to 3, a two-component relining silicone according to at least one of claims 4 to 8, and a sealing lacquer according to at least one of claims 9 or 10.

15. A relining, **characterized in that** it is prepared from a relining adhesive according to at least one of claims 1 to 3, a two-component relining silicone according to at least one of claims 4 to 8, and a sealing lacquer according to at least one of claims 9 or 10.

## Revendications

1. Adhésif de regarnissage ayant la composition suivante :
a) Composant adhésif Z, sous forme de substance pure 0 à 99% en poids,
b) Copolymère ayant comme constituent le composant adhésif Z 0 à 99% en poids
c) Copolymère dans le mélange avec les matières de départ de la synthèse provenant de la polymérisation (composant adhésif Z, monomère) 0 à 99% en poids
où la somme des proportions pondérales des constituants a), b) et c) est d'au moins 0,01% en poids, et où le mélange c) du copolymère avec les matières de départ de la synthèse provenant de la polymérisation, le composant adhésif Z et le monomère, est constitué des constituants suivants
- le copolymère 0,1 à 99,9% en poids
- le composant adhésif Z 0,1 à 99,9% en poids
- le monomère 0,1 à 99,9% en poids
où la somme des trois constituants donne 100 %,
d) Catalyseurs pour accélérer la formation du composite collé avec la silicone à coller 0 à 10% en poids
e) Solvant 1 à 99,99% en poids
f) Colorants 0 à 20% en poids
g) Tensioactif, émulsifiants et stabilisants 0 à 20% en poids
h) organohydrogénosiloxane 0 à 40% en poids
i) α,ω-divinylpolydialkylsiloxane 0 à 40 % en poids
où Z a la formule générale suivante : 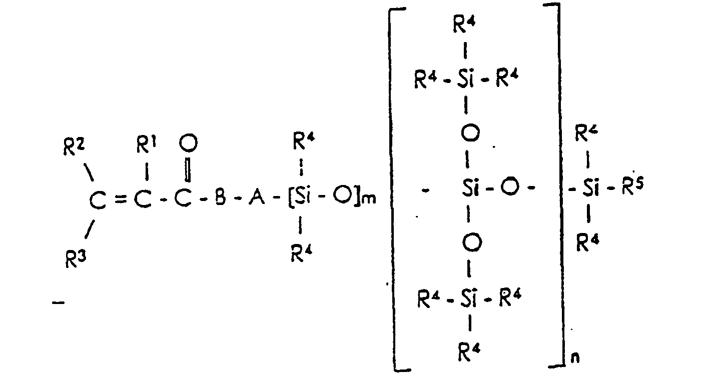 dans laquelle n et m sont des nombres entiers compris entre 0 et 1 000,
R¹ est H, un halogène, un groupe alkyle, un groupe alkyle substitué, CN,
R², R³ sont H, un halogène, un groupe alkyle, un groupe alkyl substitué et
B est O, S, NH, NR, PH, PR,
A est un radical hydrocarboné espaceur à chaîne droite ou ramifiée ayant de 0 à 30 atomes de carbone, qui peut aussi contenir un noyau cycloaliphatique ou aromatique insaturé,
R⁴ est un groupe insaturé et/ou l'un des groupes suivants: groupes alkyle, aryle, aralkyle et alkyle et aryle halogénés, cyanalkyle, cycloalkyle,
et
R⁵ peut être identique à R⁴ ou R⁶, R⁶ étant composé des motifs structuraux, à savoir
A, B, un motif polydialkylsiloxy, et le motif éthylénique insaturé :

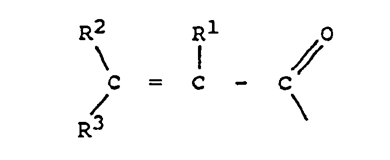

2. Adhésif de regarnissage selon la revendication 1, **caractérisé en ce que** l'on utilise comme monomères des méthacrylates et des acrylates, de préférence des méthacrylates mono ou polyfonctionnels, en particulier du méthacrylate d'alkyle, du méthacrylate de cycloalkyle, du méthacrylate d'isoalkyle, du diméthacrylate de tétraéthylèneglycol, du diméthacrylate de triéthylèneglycol, du diméthacrylate de diéthylèneglycol, du diméthacrylate d'éthylèneglycol, du diméthacrylate de polyéthylèneglycol, du diméthacrylate de butanediol, du diméthacrylate d'hexanediol, du diméthacrylate de décanediol, du diméthacrylate de dodécanediol, du diméthacrylate de bisphénol A, du triméthacrylate de triméthylolpropane, du 2,2-bis-4-(3-méthacryloxy-2-hydroxy)-phénylpropane (bis-GMA) ainsi que les produits de la réaction de di- et/ou de triisocyanates et de méthacrylates contenant des groupes OH.

3. Adhésif de regarnissage selon la revendication 1 ou 2, **caractérisé en ce que** l'on utilise comme solvant des solvants organiques tels que des composés aromatiques, des essences, des éthers, des esters, des alcools, des cétones, des aldéhydes, des amines, des acides carboxyliques, des solvants halogénés, par exemple du chloroforme et de l'eau, ainsi que toutes les combinaisons possibles des solvants mentionnés ci-dessus.

4. Silicone de regarnissage à deux composants ayant la composition suivante
a1) 0,1 à 80 % en poids d'organohydrogénopolysiloxanes ou de leurs mélanges, ayant une teneur en SiH comprise entre 2,0 et 15,0 mmol/g de SiH par rapport au polymère, avec une viscosité comprise entre 10 et 10 000 mPas,
a2) 0,1 à 80 % en poids d'organohydrogénopolysiloxanes ou de leurs mélanges, avec une teneur en SiH comprise entre 0,03 et 2,0 mmol/g de SiH par rapport au polymère, avec une viscosité comprise entre 10 et 70 000 mPas,
b) 1 à 90 % en poids d'organosiloxanes ayant deux groupes vinyle par molécule,
c) 0,0001 à 1,0 % en poids de catalyseurs, par rapport à la teneur en métal précieux pur,
d) 0 à 80 % en poids de charges de renfort,
e) 0 à 90 % en poids de charges sans effet de renfort,
f) 0 à 10 % en poids de colorants,
g) 0 à 50 % en poids d'agents liant l'humidité,
h) 0 à 70 % en poids d'organopolysiloxanes ayant plus de deux groupes vinyle par molécule,
i) 0 à 1,0 % en poids d'inhibiteurs,
j) 0 à 90 % en poids d'une résine de silicone MQ solide ou liquide,
k) 0 à 80 % en poids d'un compound,
I) 0 à 10 % en poids de tensioactifs, émulsifiants, stabilisants,
m) 0 à 90 % en poids de substances radioopaques,
n) 0 à 20 % en poids d'adsorbants de H₂ /absorbants ou de substances réduisant ou éliminant le dégagement de H₂,
o) 0 à 50 % en poids de plastifiants, d'huiles neutres et d'huiles de répartition, les organohydrogénopolysiloxanes a2) pouvant avoir la structure suivante :
A. formule développée des organohydrogénosiloxanes linéaires 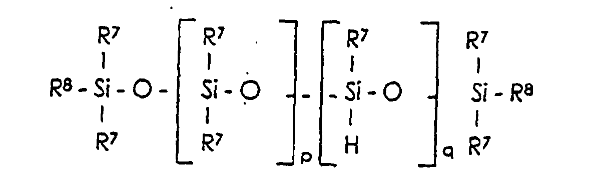 avec p = 0 à 1 500
avec q = 1 à 1 500
avec R⁷ = un groupe alkyle, aryle, aralkyle et alkyle et aryle halogénés, cyanalkyle, cycloalkyle et cycloalcényle
avec R⁸ = H

5. Silicone de regarnissage à deux composants selon la revendication 4 dans laquelle les organohydrogènopolysiloxanes a1) ont la structure suivante :
A. formule développée des organohydrogénosiloxanes linéaires 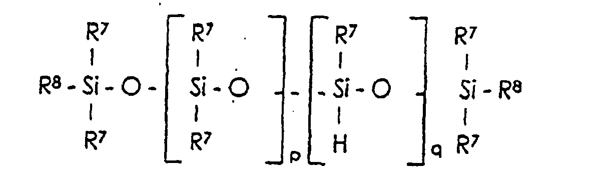 avec p = 0 à 1 500
avec q = 0 à 1 500
avec R⁷ = un groupe alkyle, aryle, aralkyle et alkyle et aryle halogénés, cyanalkyle, cycloalkyle et cycloalcényle
avec R⁸ = R⁷, H
B les résines MQ contenant des SiH se composant de motifs (R⁷)₃SiO_{1/2}, (R⁷)₂(H)SiO_{1/2} et SiO_{4/2}, où on peut encore avoir aussi comme motifs T le motif trifonctionnel R⁸SiO_{3/2} et en tant que motifs D le motif bifonctionnel R⁷R⁸SiO_{2/2},
où R⁷ et R⁸ ont les significations données en A.

6. Silicone de regarnissage à deux composants selon la revendication 4 ou 5, **caractérisée en ce que** les organopolysiloxanes vinylés ayant deux groupes vinyle par molécule ont la formule générale
CH₂=CH-SiR₂O-(SiR₂O)n-SiR₂-CH=CH₂
dans laquelle
R est un groupe alkyle, aralkyle, et alkyle et aryle halogénés, cyanalkyle, cycloalkyle et cycloalcényle, et
n est un nombre entier valant de 21 à 1 500, où "n" représente des valeurs calculées par la théorie, qui ont été calculés à partir de la teneur en groupes vinyle déterminée expérimentalement, dans l'hypothèse que chaque molécule contient deux groupes vinyle et que R est le groupe méthyle.

7. Silicone de regarnissage à deux composants selon l'une des revendications 4 à 6, **caractérisée en ce que** le catalyseur est un sel, un complexe ou une forme colloïdale des métaux de transition du huitième groupe secondaire.

8. Silicone de regarnissage à deux composants selon l'une des revendications 4 à 7, **caractérisée en ce que** les charges d) de la silicone de regarnissage sont rendues hydrophobes ou sont hydrophiles et/ou **en ce que** les charges d) et/ou e) de la silicone de regarnissage ont subi un traitement de surface, où on a utilisé des silanes et/ou des acides gras comportant des groupes fonctionnels à même de réagir avec les composants a), b) et c).

9. Vernis de scellement à deux composants, dans lequel les deux composants subissent une réticulation chimique par l'intermédiaire d'une réaction d'hydrosilylation catalysée par un métal précieux, pour lesquels il s'agit d'un organohydrogénopolysiloxane ayant une teneur élevée en SiH comprise entre 2,0 et 15 mmol/g par rapport au polymère et d'un organohydrogénopolysiloxane ayant une faible teneur en SiH comprise entre 0,05 et 2 mmol/g par rapport au polymère, le vernis de scellement ayant une viscosité comprise entre 25 et 10 000 mPas, le vernis de scellement à deux composants ayant les mêmes constituants que la silicone de regarnissage à deux composants conforme à l'une des revendications 4 à 8, lesquels peuvent être présents dans les mêmes proportions en pourcentage.

10. Vernis de scellement à deux composants selon la revendication 9, **caractérisé en ce que** le vernis de scellement à deux composants est présent dans une cartouche.

11. Procédé de fabrication d'un regarnissage direct, constitué selon l'une des revendications 1 à 7, **caractérisé en ce que**
- on nettoie et/ou rend rugueux le plastique de prothèse qui doit être pourvu d'un regarnissage,
- puis on applique l'adhésif à un composant conforme à l'une des revendications 1 à 3 sur une épaisseur de couche de 0,1 à 500 µm,
- éventuellement, on procède ensuite à un étuvage à une température supérieure à 30 °C pendant au moins 5 minutes,
- après élimination du solvant par évaporation, on applique la silicone de regarnissage à deux composants conforme à l'une des revendications 4 à 8,
- pendant la durée de l'utilisation de la silicone de regarnissage, on procède à une prise d'empreinte fonctionnelle,
- après durcissement de la silicone de regarnissage, on réalise un usinage par taille ou fraisage, et **en ce que** les rugosités de surface qui se forment sous l'effet de l'usinage sont scellées avec du vernis de scellement conforme à la revendication 9 sur une épaisseur de couche de 0,1 à 1 000 µm.

12. Procédé de fabrication d'un regarnissage indirect, constitué selon l'une des revendications 1 à 7, **caractérisé en ce que**
- on nettoie et/ou rend rugueux le plastique de prothèse qui doit être pourvu d'un regarnissage,
- puis on applique l'adhésif à un composant conforme à l'une des revendications 1 à 3 sur une épaisseur de couche de 0,1 à 500 µm,
- éventuellement, on procède ensuite à un étuvage à une température supérieure à 30 °C pendant au moins 5 minutes,
- après élimination du solvant par évaporation, on applique la silicone de regarnissage à deux composants conforme à l'une des revendications 4 à 8,
- après le durcissement de la silicone de regarnissage, on réalise un usinage par taille ou fraisage,
- on scelle avec le vernis de scellement conforme à la revendication 9, sur une épaisseur de couche de 0,1 à 1 000 µm, les rugosités de surface qui se sont formées sous l'effet de l'usinage.

13. Procédé selon l'une des revendications 11 ou 12, **caractérisé en ce que** l'adhésif conforme au moins à l'une des revendications 1 à 3 est utilisé sous forme liquide ou dissoute.

14. Système de regarnissage, **caractérisé en ce qu'**il contient un adhésif de regarnissage conforme au moins à l'une des revendications 1 à 3, une silicone de regarnissage à deux composants conforme au moins à l'une des revendications 4 à 8 et un vernis de scellement conforme au moins à l'une des revendications 9 ou 10.

15. Regarnissage, **caractérisé en ce qu'**il contient un adhésif de regarnissage conforme au moins à l'une des revendications 1 à 3, une silicone de regarnissage à deux composants conforme au moins à l'une des revendications 4 à 8 et un vernis de scellement conforme au moins à l'une des revendications 9 ou 10.
